# EUROPEAN PATENT APPLICATION

(11) **EP 4 603 070 A2**
(43) Date of publication of application: **20.08.2025**
(21) Application number: 25187351.9
(22) Date of filing: 12.01.2024
(51) Int. Cl.: A61F 5/445

(54) **AN OSTOMY APPLIANCE**

(30) Priority: 12.01.2023 US 202363438673 P; 12.01.2023 US 202363438678 P; 12.01.2023 US 202363438684 P; 22.02.2023 GB 202302546; 22.02.2023 GB 202302547; 22.02.2023 GB 202302548
(62) Divisional of application: 24705029.7
(71) Applicant: ConvaTec Technologies Inc., Las Vegas, NV 89169-6754 (US)
(72) Inventor: CHEN, Hung-Hsiang, Las Vegas, 89169-6754 (US); DAVIS, Jeremy Parker, Las Vegas, 89169-6754 (US); CHEN, Chih-Ho, Las Vegas, 89169-6754 (US)
(74) Representative: Wilson Gunn

(57) **Abstract**

An ostomy appliance comprises a pouch and a drain. The drain is formed of two walls attached to one another lateral edges to define a channel therebetween and comprises pursing strips to separate the walls when compressed in a direction perpendicular to the lateral edges of the drain. A pursing strip comprises a transverse edge perpendicular to the lateral edges and the transverse edge comprises a notch spanning at least half of the transverse edge. A pursing strip may comprise a line of weakness. The line of weakness may span between side edges of the pursing strip. The line of weakness may be curved. A pursing strip may comprise two converging lines of weakness spanning between upper and lower edges of the pursing strip. The drain may comprise two or three pursing strips. A gap may be provided between pursing strips.

## Description

### Technical Field of the Invention

The present invention relates to ostomy appliances, and in particular drainable ostomy appliances, and most particularly ostomy appliances with drains that can be opened and resealed by the user.

### Background to the Invention

Ostomy appliances are used by people with a stoma to capture stomal output that passes out fo the stoma such that it can be conveniently and hygienically disposed of. To reduce the number of times that a person needs to replace an ostomy appliance, drainable (or "open") ostomy appliances have been developed that have a drain which can be opened to allow the contents of the ostomy appliance to be disposed of. The drain can then be re-sealed to allow continued use of the ostomy appliance which has corresponding cost savings as well as being more convenient for the user and environmentally friendly.

The addition of a drain as described above can however lead to some problems with use of the ostomy appliance. For example, if the user is not able to securely reseal the drain then there is an increased likelihood of the contents of the ostomy appliance leaking through the drain which can lead to a hygienic risk and an unpleasant smell as well as discomfort and embarrassment for the user. **In** addition, through multiple use cycles, the opposing internal walls of the drain are likely to become wet. This makes opening of the drain more difficult as the flexible plastics material typically used sticks to itself when wet.

To overcome the above issues, drainable ostomy appliances have been developed with one or more pursing strips provided on the drain to provide a rigid structure the drain. The drain can then be folded around the pursing strips which improves the re-sealability of the drain. The pursing strips can also be used to assist with opening of the drain, for example in US8672907B2 a pair of pursing strips are provided with intrinsic curvature to separate the drain, however, this intrinsic curvature adds additional size to the drain which could be uncomfortable for the user.

**In** a different example US8672907B2 discloses a small notch provided in a pursing strip and also in a corresponding position on the drain to allow the user to insert their finger into the drain to open it. Howver, this is likely to be unpleasant for the user and could also present a hygienic risk as the drain is typically not clean after one use cycle and even on the first use they would need to both open the drain to let stomal output flow out the drain and remove their finger before the liquids in the stomal output got down to their finger, which is likely to be difficult.

**In** another example US2019/0328572A1 discloses a drain with axially and transversely extending fold-lines covering one side of the drain to guide the opening and closing of the drain. **In** one embodiment, the axially extending fold lines are slanted so that they converge at a distal end of the drain. Howver, this is likely to be unpleasant for the user because of the additional bulk that is present in the drain due to the additional rigidity added over all of one side of the drain. In addition, this additional bulk means the drain is not well suited to being folded back onto itself and so an optional stiffening is provided on the other side of the fold-lines at the distal end of the drain to allow the flexible drain material to be folded around something. Of course, this optional stiffening adds further bulk to the drain exacerbating any discomfort due to the size of the drain.

It is therefore an object of the present invention to provide an ostomy appliance that at least partially addresses the above problems.

### Summary of the Invention

In broad terms, the invention concerns a pursing strip incorporating a notch. The pursing strip may comprise a transverse edge. The notch may span at least half of the transverse edge. The pursing strip may be comprised in a drain of an ostomy appliance. The ostomy appliance may comprise a pouch and a drain. The drain may be formed of two walls, for example proximal and distal walls. The two walls may be attached to one another along two lateral edges of the drain to define a channel therebetween. The drain may comprise one or more of the pursing strips. The one or more pursing strips may be configured to separate the walls of the drain when compressed in a direction perpendicular to the two lateral edges of the drain. At least one of the one or more pursing strips may be a first pursing strip. The first pursing strip may be the pursing strip as outlined above, i.e. it may comprise a notch. The first pursing strip may comprise an upper and a lower edge which may be substantially perpendicular to the two lateral edges of the drain. The lower edge may be the transverse edge outlined above. The transverse edge may therefore be substantially perpendicular to the two lateral edges of the drain.

According to a first aspect of the present invention, there is provided an ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a pursing strip of the one or more pursing strips comprises a transverse edge substantially perpendicular to the two lateral edges of the drain and the transverse edge comprises a notch spanning at least half of the transverse edge.

The inventors have found that by providing a notch that spans at least half of the width of a pursing strip provides the pursing strip with mechanical properties that enable it to effectively separate the walls of the drain and open the drain when compressed. As the notch is provided along one transverse edge, this side of the pursing strip is both weaker and has more space to flex as compared to the other transverse edge. So when compressed, the pursing strip has a varying curvature in a direction parallel to the lateral edges which causes the two walls to separate. In addition, once the compression is released, the pursing strip is not damaged and can recover a flat shape to allow the drain to be efficiently closed without adding additional bulk to the ostomy appliance.

Each of the one or more pursing strips may be planar. Each of the one or more pursing strips may be substantially flat (at rest). Each of the one or more pursing strips may have an intrinsic curvature of approximately zero (at rest). The intrinsic curvature of each of the one or more pursing strips may be approximately zero as measured in any plane perpendicular to the walls of the drain. Thus, at rest, i.e. in the absence of a compressive force perpendicular to the walls of the drain, the pursing strips are not bent and therefore do not project out of the plane of the walls to cause potential discomfort and inconvenience to the user during use.

The drain may have a width measured between the lateral edges of the drain. Each of the one or more pursing strips may have a width substantially parallel to the width of the drain. The width of the drain may be the same, or substantially the same, as the width of the pursing strip. Thus, the pursing strips may be used to effectively seal the drain as it spans all the way across the width of the drain.

Each of the one or more pursing strips may have two side edges corresponding to the lateral edges of the drain. Each of the one or more pursing strips may have a length defined parallel to their two side edges. The length of a pursing strip may be the same as the length of a side edge. Each of the one or more pursing strips may have an upper edge and a lower edge. Thus, each of the one or more pursing strips may be substantially rectangular.

Each of the one or more pursing strips may be more rigid than the walls of the drain. Each of the one or more pursing strips may comprise a strip of flexible material attached to a wall of the drain. Each of the one or more pursing strips may be formed from a flexible plastics material, such as high density polyethylene (HDPE), or polystyrene. Each of the walls may be formed from a flexible plastics material, such as low density polyethylene (LDPE) or ethylene-vinyl acetate (EVA). Thus, the pursing strips provide increased resilience to the drain and can be used to effectively open and seal the drain.

The drain may comprise a proximal end to receive stomal output from the pouch. The distal end may be at an opposite end of the drain from the proximal end when the drain is in an open, or "unfurled" configuration. The drain may have a length measured between the proximal and distal ends of the drain. The pursing strip may be arranged at the distal end of the drain. The notch may be provided in a distal transverse edge. The distal transverse edge may be arranged at the distal end of the drain, Thus, the pursing strip can be used to separate the walls of the drain at the end of the drain to allow better control of emptying the pouch.

The notch may have a width parallel to the transverse edge. The notch may have a length perpendicular to its width. The length of the notch may be in a plane defined by the pursing strip. The notch may have an aspect ratio (width:length) of at least 5:1, 7:1, 10:1, or 12:1. The notch may have an aspect ratio (width:length) of no more than 15:1, 12:1, 10:1, or 7:1. Preferably, the notch has an aspect ratio (width:length) of 12:1. Thus, the notch has an aspect ratio that optimises the mechanical properties of the pursing strip to help with opening of the drain without significant risk of damage to the pursing strip during bending.

The notch may be curved. The notch may be arced. The shape of the notch may be defined by an arc. The arc may have a radius of curvature that is greater than the width of the notch. The arc may have a radius of curvature that is greater than a width of the pursing strip, for example about 50% greater than the width of the pursing strip. The arc may have a radius of curvature that is greater than a width of the drain. Thus, the arced shape helps to ensure the notch deforms predictably to open the drain.

The pursing strip may have a length parallel to the length of the notch. The length of the notch may be less than half, a third or a quarter of the length of the pursing strip. The length of the notch may at least a fifth or a quarter of the length of the pursing strip. Preferably, the length of the notch is about a quarter the length of the pursing strip. Thus, the length of the notch is chosen to provide adequate deformation of the pursing strip with risking damage of the pursing strip.

The notch may span at least 40%, 50%, 60% or 80% of the transverse edge. The notch may span no more than 95%, 90%, 80% or 60% of the transverse edge. Preferably, the notch spans no more than 90% of the transverse edge, for example the notch may span 60% to 90% of the transverse edge. The notch may span 85% of the transverse edge. Thus, a section of the transverse edge is free of the notch and ensures the pursing strip retains some mechanical strength to support deformation and opening of the drain.

The notch may be arranged towards a midpoint along the transverse edge. Preferably, the notch is arranged at the midpoint along the transverse edge. An apex of the arc of the notch may be arranged at or towards the midpoint along the transverse edge. The notch may be symmetrical about a midline of the pursing strip (the midline expending perpendicular to the transverse edge). Thus, the notch is centrally located to ensure even and predictable deformation of the drain during opening.

The parts of the transverse edge adjacent the side edges may not comprise the notch. Thus, compression of the pursing strip is easier.

The distal end of the drain may be straight. The notch may expose an end region of the distal end of the drain. The end region may not be directly attached to the one or more pursing strips. Thus, the end region provides further variation in the mechanical strength of the respective parts of the drain which increase the likelihood of separation of the walls of the drain during compression of the pursing strips.

The drain may have closed configuration in which passage of stomal output through the drain is inhibited. The drain may be rolled and/or folded in the closed configuration. The drain may define a tortuous channel in the closed configuration. The drain may be folded about the, or each, pursing strip in the closed configuration. Each pursing strip may define the locations and orientations of one or more folds of the drain. The drain may be foldable about one or more fold lines, or folds. One or more of the folds may close the tortuous channel. Thus, the pursing strip(s) may allow the drain to be effectively sealed shut.

The drain may have open configuration in which passage of stomal output through the drain is permitted. In the open configuration, the drain may be unfurled, e.g. unrolled and unfolded. The drain may define a substantially linear channel in the open configuration. Thus, the drain may be placed in the open configuration to allow draining of the pouch's contents.

The pouch may be formed from two walls sealed about their periphery. The two walls of the pouch may be formed from the same pieces of material as the corresponding two walls (i.e. the proximal and distal walls) of the drain. The two walls of the pouch may comprise the proximal wall and the distal wall of the drain. The pouch and drain may therefore be integrally formed. The proximal wall intended to be closest to the body of the user in use. The distal wall may be on an opposite side of the pouch and/or drain to the proximal wall.

The first pursing strip may be arranged on the proximal wall of the drain. The first pursing strip may be the only pursing strip arranged on the proximal wall of the drain. The one or more pursing strips may comprise a second pursing strip. The second pursing strip may be arranged on the distal wall. The second pursing strip may be the only pursing strip arranged on the distal wall of the drain. A lateral gap may be provided between the upper edge of the first pursing strip and a lower edge of the second pursing strip. The lateral gap may be at least 1, 2, 3, 4 or 5 mm. The lateral gap may be sized to allow the formation of a fold in the drain between the first and second pursing strips. Thus, the bulk of the drain is minimised without compromising performance.

The pouch may comprise a closure mechanism configured to retain the drain in the closed configuration. The closure mechanism may comprise one or more fastening elements. The one or more fastening elements may comprise a pouch fastening element provided on the pouch. The pouch fastening element may be positioned adjacent to the drain. The pouch fastening element may be configured to attach to the drain to retain it in the closed configuration. The pouch fastening element may be provided on the distal wall. Thus, the pouch fastening element is easily accessible to the user.

In the closed configuration, the drain may be folded about the first pursing strip at least three times. Thus, a fluid tight seal is generated by folding the drain.

The closure mechanism may comprise a closure flap. The closure flap may be provided on the pouch adjacent to the drain. The closure flap may be provided on distal wall. The closure flap may be configured to overlie the drain when the drain is in the closed configuration. The pouch fastening element may be provided on a closure flap. Thus, the closure flap can be used to more securely seal the drain.

The one or more fastening elements may comprise a drain fastening element provided on the drain. The drain fastening element may be positioned towards the proximal end of the drain. The drain fastening element may be configured to fasten to the pouch to retain the drain in the closed configuration. Thus, the drain may be retained in the closed configuration by a fastening element positioned on the drain.

The one or more fastening elements may comprise two fastening elements configured to fasten to one another. The two fastening elements may comprise corresponding complementary fastening surfaces. The fastening surfaces may comprise any of: hook and loop fasteners; hook and hook fasteners; and mushroom-head fasteners. The two fastening elements may comprise the pouch fastening element and drain fastening element as described above.

The drain may be provided at a bottom of the ostomy appliance. The pouch may comprise a stomal inlet configured to allow stomal output to pass from the user's stoma and into the pouch. The proximal wall may comprise the stomal inlet. The stomal inlet may be provided towards a top of the ostomy appliance. The bottom of the ostomy appliance may be at or towards the lowest point of the ostomy during use. Stomal output stored in the pouch may flow out of the drain under the action of gravity. The top of the ostomy appliance may be at or towards an opposite end of the ostomy appliance from the bottom.

The pouch may comprise an adhesive collar configured to attach the pouch to the body of the user. The adhesive collar may be generally annular in shape and provided around the stomal inlet. The adhesive collar may be provided on the proximal wall. The adhesive collar may be formed of a mouldable adhesive material, for example a hydrocolloid adhesive. The adhesive collar may be provided with a release liner to protect the adhesive collar prior to use.

The pouch may comprise a gas vent configured to allow gases in the pouch to leave the pouch. The gas vent may be provided in the distal wall of the pouch. The gas vent may be provided in register with the stomal inlet.

Each of the one or more pursing strips may have substantially the same width. Each of the one or more pursing strips may have substantially the same length. This helps to ensure a better sealing action.

The first and second pursing strips may have equal widths. The first and second pursing strips may have equal lengths. The second pursing strip may not comprise a line of weakness. Alternatively, the second pursing strip may also comprise a line of weakness, or at least two lines of weakness which may have any of the optional features set out above. Thus, the first and second pursing strips can be used to effectively seal the drain as they are the same length and width.

The lateral gap may be free of pursing strips. Thus, the drain may be folded to place the first pursing strip and second pursing strip on top of one another to create a tighter seal when the drain is in the closed configuration.

The one or more pursing strips may comprise a line of weakness. One or more of the one or more pursing strips may each comprise a line of weakness. The first pursing strip may comprise a line of weakness. Where the first pursing strip comprises a line of weakness, it may not comprise a notch as described above.

The second pursing strip may have substantially constant mechanical properties. The second pursing strip may not comprise a line of weakness. Thus, the second pursing strip has a constant rigidity and is therefore able to securely seal the drain.

Where there are two or more pursing strips, at least two of them may have unequal widths. The drain may comprise a narrow pursing strip that has a smaller width than the first pursing strip. The narrow pursing strip may have a width about 10-30% less than the width of the first pursing strip. Each of the one or more pursing strips may have a width that is no larger than the width of the drain. Two of the two or more pursing strips may each be arranged at the distal end of the drain. The narrow pursing strip may be arranged at the distal end of the drain. The narrow pursing strip may be arranged on the distal wall of the drain. The narrow pursing strip may be a second or third pursing strip, or the weakened pursing strip (see below), depending on the configuration of the drain. Thus, the unequal widths may be used to increase the propensity for the drain to open because one flexes before the other during compression.

The ostomy appliance may comprise only the first pursing strip and the second pursing strip (i.e. the ostomy appliance may comprise no further pursing strips).

The one or more pursing strips may comprise a weakened pursing strip. The weakened pursing strip comprises one or more lines of weakness.

The weakened pursing strip may be a second pursing strip where there are two or more pursing strips.

The ostomy appliance may comprise three or more pursing strips. The weakened pursing strip may be a third pursing strip where there are three or more pursing strips.

The weakened pursing strip may have any one or more of the features of the one or more pursing strips, first pursing strip and/or second pursing strip as outlined above. Where a pursing strip comprises a line of weakness, it may be referred to as a weakened pursing strip, or just a pursing strip, interchangeably. The weakened pursing strip may have side, upper and lower edges. The transverse edge may be the lower edge of the weakened pursing strip.

The weakened pursing strip may be positioned adjacent to the distal end of the drain. The weakened pursing strip may be positioned on the opposite side of the drain to the first pursing strip. The weakened pursing strip may be positioned on the distal wall of the ostomy appliance.

The weakened pursing strip may be positioned on the proximal wall of the ostomy appliance, especially where the weakened pursing strip is the first pursing strip. A narrow pursing strip may be provided on the distal wall where the weakened pursing strip is provided on the proximal wall.

The, or each, of the lines of weakness may comprise a groove. Each pursing strip may comprise an outside surface. The outside surface may be distal from the two walls of the drain. Each groove may be formed in the outside surface of the weakened pursing strip. Thus, each groove allows the weakened pursing strip to bend more easily without breaking and also while reducing the risk of damage to the walls of the drain by pinching. Each groove may have a width measured perpendicular to a respective line of weakness. The width of each groove may be about 0.25 mm.

Each, or a, line of weakness may comprise one or more perforations.

Each, or a, line of weakness may be at least partially straight, or they may be substantially straight, or completely straight. Thus, the line of weakness has a simple but effective straight shape.

The whole of any one or more of the one or more pursing strips may be attached to a wall of the drain. Thus, the wall matches the shape of each such pursing strip to help ensure it can separate from the other wall.

The one or more lines of weakness may comprise a longitudinal line of weakness that spans the majority of the way, or more preferably all the way from the upper edge to the lower edge of the weakened pursing strip. Thus, the weakened pursing strip can more effectively and predictably separate the walls of the drain.

The one or more lines of weakness may comprise two converging lines of weakness. The first pursing strip may comprise the two converging lines of weakness.

According to another aspect of the invention, there is provided an ostomy appliance comprising a pouch and a drain, the drain formed of proximal and distal walls attached to one another along two lateral edges of the drain to define a channel therebetween, the drain comprising a distal end for output of stomal effluent from the pouch, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip. The lateral gap described above may provide the longitudinal gap.

The inventors have found that by providing lines of weakness that are converging provides the weakened pursing strip with mechanical properties that enable it to effectively separate the walls of the drain and open the drain when compressed. This is because the converging lines create an imbalance, because when compressed, the weakened pursing strip flexes with a varying angle that encourages the two walls to separate

Each of the converging lines of weakness may be a longitudinal line of weakness. Thus, the one or more lines of weakness may comprise two longitudinal lines of weakness that are converging. The converging lines of weakness may be arranged to converge at a point that is not on the weakened pursing strip. Consequently, the converging lines of weakness are converging but do not converge on the weakened pursing strip. This helps to ensure that there is always a gap between the converging lines of weakness which allows the weakened pursing strip to flex out of the plane of the drain more easily.

The two converging lines of weakness may be separated by at least one quarter or one third of the width of the drain. Preferably the two converging lines of weakness may be separated by at least one half of the width of the drain. This helps ensure the central region of the drain is generally flat and able to move uniformly out of the plane of the drain to open the drain.

A point along each of the converging lines of weakness may correspond to a midpoint along the length of the weakened pursing strip. A midpoint along each of the converging lines of weakness may correspond to a midpoint along the length of the weakened pursing strip.

The shape of each of the converging lines of weakness may be asymmetric about a line connecting the two side edges. The shape of each of the converging lines of weakness may be asymmetric about a line perpendicular to the two side edges. The converging lines of weakness may be a mirror image of one another about a line extending midway between the two side edges from the upper edge to the lower edge. The converging lines of weakness may be a mirror image of one another about a line parallel the two side edges. Thus, the converging lines of weakness are shaped to ensure asymmetric bending of the weakened pursing strip to open the drain.

The converging lines of weakness may be arranged at an angle of 5-40 degrees from a lateral edge of the drain. The converging lines of weakness may be arranged at an angle of at least 5, 10, 15 or 20 degrees from, or with respect to, a lateral edge of the drain. The converging lines of weakness may be arranged at an angle of no more than 40, 35, 30, 25 or 20 degrees from a lateral edge of the drain. Preferably, the converging lines of weakness may be arranged at an angle of about 15 degrees from a lateral edge of the drain.

The converging lines of weakness may be arranged at an angle of 10 to 40 degrees with respect to one another. The converging lines of weakness may be arranged at an angle of at least 5, 10, 15, 20, 25, or 30 degrees with respect to one another. The converging lines of weakness may be arranged at an angle of no more than 45, 40, 35 or 30 degrees with respect to one another. Preferably, the converging lines of weakness are arranged at an angle of 30 degrees with respect to one another. Thus, the converging lines of weakness are optimally angled to ensure efficient opening of the drain.

The two converging lines of weakness may diverge in a direction towards the distal end of the drain. The two converging lines of weakness may diverge in a direction away from the proximal end of the drain. The two converging lines of weakness may converge in a direction towards the proximal end of the drain. The two converging lines of weakness may converge in a direction away from the distal end of the drain. The lower edge of each of the one or more pursing strips may be closer to the distal end of the drain than the upper edge. The two converging lines of weakness may be closer to one another at the upper edge of the weakened pursing strip than at the lower edge of the weakened pursing strip. Thus, this helps to ensure the distal end of the drain is opened widest to allow efficient passage of stomal output out of the drain.

Accordingly in one aspect there is provided an ostomy appliance comprising a pouch and a drain, the drain formed of proximal and distal walls attached to one another along two lateral edges of the drain to define a channel therebetween, the drain comprising a distal end for output of stomal effluent from the pouch, the drain comprises one or more pursing strips configured to separate the walls of the drain when compressed in a direction perpendicular to the lateral edges of the drain; wherein a first pursing strip of the one or more pursing strips comprises an upper edge and a lower edge both perpendicular to the lateral edges and two converging lines of weakness spanning between the upper and lower edges; wherein two lines of weakness converge in a direction away from the distal end of the drain.

The two converging lines of weakness may converge in a direction towards the distal end of the drain. The two converging lines of weakness may diverge in a direction towards the proximal end of the drain. The two converging lines of weakness may be closer to one another at the lower edge of the weakened pursing strip than at the upper edge of the weakened pursing strip.

Each of the two converging lines of weakness may be arranged in a respective (lateral) half of the weakened pursing strip. Each of the one or more pursing strips may have a width measured between the side edges of the pursing strip. Each of the two converging lines of weakness may cover no more than 25%, 20%, 15%, or 10% of the width of the weakened pursing strip. Each of the two converging lines of weakness may cover at least 5%, 10%, 15%, or 20% of the width of the weakened pursing strip. Preferably, each of the two converging lines of weakness may cover about 10% of the width of the weakened pursing strip. Thus, the line of weakness does not cause excessive changes to the structural properties of the weakened pursing strip which could lead to poor sealing performance of the drain.

The one or more lines of weakness may comprise a transverse line of weakness that is arranged, extending and/or spanning between the side edges of the weakened pursing strip. The one or more lines of weakness may be at least partly curved, or substantially curved or completely curved. The transverse line of weakness may be curved.

Thus, in one aspect there is provided an ostomy appliance comprising a pouch and a drain, wherein the drain is formed of two walls attached to one another along two lateral edges of the drain to define a channel therebetween and the drain comprises one or more pursing strips configured to separate the walls of the drain when compressed in a direction perpendicular to the two lateral edges of the drain; wherein a pursing strip of the one or more pursing strips comprises two side edges corresponding to the two lateral edges of the drain and a line of weakness spanning between the side edges, and the line of weakness is curved.

The inventors have found that by providing a transverse line of weakness that is curved and spans between the side edges provides the weakened pursing strip with mechanical properties that enable it to effectively separate the walls of the drain and open the drain when compressed. This is because the curvature creates an imbalance as the parts of the weakened pursing strip on either side of line of weakness have different mechanical properties and furthermore, the mechanical properties of each side varies along the line of weakness between the side edges. So when compressed, the weakened pursing strip flexes with a varying curvature that encourages the two walls to separate.

The first pursing strip may comprise the transverse line of weakness.

The transverse line of weakness may extend from a point along one of the two side edges that is offset from a midpoint along the length of the side edge. The transverse line of weakness may extend from a point along each of the two side edges that is offset from a midpoint along the length of the corresponding side edge. The transverse line of weakness may extend from the same point along each of the two side edges (i.e. if the midpoint of one side edge, then the midpoint of the opposing side edge, and if a point that is offset from a midpoint along the length of one side edge, then a correspondingly offset point along the length of the opposing side edge). The transverse line of weakness may span the majority of the way, or more preferably all the way from one side edge to the opposing side edge. Thus, the weakened pursing strip can more effectively and predictably separate the walls of the drain.

The transverse line of weakness may be arranged in a half of the pursing strip distal from the distal end of the drain. The midpoint along the transverse line of weakness may be the closest point on the line of weakness to the distal end of the drain. The ends of the transverse line of weakness, which may be positioned at the side edges of the pursing strip, may be the furthest points on the transverse line of weakness from the distal end of the drain. Thus, the midpoint may flex more than the ends to allow the walls to separate.

The length of the weakened pursing strip may be the same as the length of a side edge. A point along the transverse line of weakness may correspond to a midpoint along the length of the weakened pursing strip. A midpoint along the transverse line of weakness may correspond to a midpoint along the length of the weakened pursing strip. Thus, the transverse line of weakness is shaped to encourage bending at the centre of the weakened pursing strip but preferably not at the centre along its length at the side edges to help create non-uniform bending and separation of the walls.

The shape of the transverse line of weakness may be asymmetric about a line connecting the two side edges. The shape of the transverse line of weakness may be asymmetric about a line perpendicular to the two side edges. The shape of the transverse line of weakness may be symmetric about a line extending midway between the two side edges. The shape of the transverse line of weakness may be symmetric about a line parallel the two side edges. Thus, the transverse line of weakness is shaped to ensure asymmetric bending of the weakened pursing strip to open the drain.

The transverse line of weakness may be arranged in one half of the weakened pursing strip, that is it may extend in one half of the pursing strip. The other half of the pursing strip may be free of lines of weakness. The transverse line of weakness may span less than half of the length of the weakened pursing strip, or less than a third of the length of the weakened pursing strip, or less than a quarter of the length of the weakened pursing strip. Thus, the transverse line of weakness does not cause excessive changes to the structural properties of the weakened pursing strip which could lead to poor sealing performance of the drain.

The transverse line of weakness may be arced. The transverse line of weakness may define an arc. The arc may have a radius of curvature that is greater than the width of the weakened pursing strip. The arc may have a radius of curvature that is at least two times greater than the width of the weakened pursing strip. Thus, the transverse line of weakness has a simple but effective arced shape.

The transverse may be convex on a side facing the distal end of the drain. This can help to preferentially open the distal end as the section of pursing strip in the midpoint across the distal end is thinner and so easier to bend.

The curvature of the notch and line of weakness may be opposing along the length of the drain. A first pursing strip comprising a notch may be arranged on one side of the drain. A second pursing strip comprising a curved line of weakness may be arranged on the other side of the drain. The first and second pursing strips may be arranged at the same point along the length of the drain. The notch and line of weakness may not overlap. Thus, the opposing curvature and respective positions of the notch and line of weakness helps the drain to open more effectively.

The line of weakness may be curved such that folding of the pursing strip and/or drain about the line of weakness is inhibited. The line of weakness may resist folding of the pursing strip and/or drain by at least 45 degrees, at least 90 degrees, at least 120 degrees, or at least 150 degrees about the line of weakness. The drain may not be foldable about the curved line of weakness. The line of weakness may be provided on one side of at least one of the one or more fold lines of the drain. The line of weakness may be provided between adjacent fold lines of the drain. Thus, the curvature is designed to help the drain open and not to allow folding of the drain about the line of weakness.

The optional features of the line of weakness, transverse line of weakness, longitudinal line of weakness and converging lines of weakness may equally apply to any one line of weakness, such as the transverse line of weakness, longitudinal line of weakness, or a or each of the converging lines of weakness. Thus, the invention provides for flexible design of a weakened pursing strip depending on the requirements of a specific ostomy appliance. Similarly, the features of any aspect defined herein may apply to an ostomy appliance, ostomy drain or pursing strip as described herein. Certain non-limiting preferred embodiments and combinations of features are additionally described below.

In one preferred embodiment, there is provided an ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a pursing strip of the one or more pursing strips comprises a transverse edge substantially perpendicular to the two lateral edges of the drain and the transverse edge comprises a notch spanning at least half of the transverse edge, wherein the notch has a width parallel to the transverse edge and a length perpendicular to its width and in a plane defined by the pursing strip, wherein the notch has an aspect ratio (width:length) of at least 7:1.

In one preferred embodiment, there is provided an ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a pursing strip of the one or more pursing strips comprises a transverse edge substantially perpendicular to the two lateral edges of the drain and the transverse edge comprises a notch spanning at least half of the transverse edge, wherein the notch is arced and the arc radius of curvature is greater than the width of the notch.

In one preferred embodiment, there is provided an ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a pursing strip of the one or more pursing strips comprises a transverse edge substantially perpendicular to the two lateral edges of the drain and the transverse edge comprises a notch spanning at least half of the transverse edge, wherein the notch spans at least 60% of the transverse edge and preferably no more than 90% of the transverse edge.

In one preferred embodiment, there is provided an ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a first pursing strip of the one or more pursing strips comprises a transverse edge substantially perpendicular to the two lateral edges of the drain and the transverse edge comprises a notch spanning at least half of the transverse edge, and the one or more pursing strips comprises a narrow pursing strip that has a smaller width than the first pursing strip.

In one preferred embodiment, there is provided an ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a first pursing strip of the one or more pursing strips comprises a transverse edge substantially perpendicular to the two lateral edges of the drain and the transverse edge comprises a notch spanning at least half of the transverse edge, and the one or more pursing strips comprises a weakened pursing strip comprising a curved line of weakness, preferably wherein the notch is arced and the curvature of the arc and line of weakness are opposing along the length of the drain.

In one preferred embodiment, there is provided an ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a pursing strip of the one or more pursing strips comprises two side edges corresponding to the two lateral edges of the drain and a line of weakness spanning between the side edges, and the line of weakness is curved, wherein the line of weakness extends from a point along one of the two side edges that is offset from a midpoint along the length of the side edge.

In one preferred embodiment, there is provided an ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a pursing strip of the one or more pursing strips comprises two side edges corresponding to the two lateral edges of the drain and a line of weakness spanning between the side edges, and the line of weakness is curved, wherein the line of weakness is arranged in one half of the pursing strip, preferably only one half.

In one preferred embodiment, there is provided an ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a pursing strip of the one or more pursing strips comprises two side edges corresponding to the two lateral edges of the drain and a line of weakness spanning between the side edges, and the line of weakness is curved and comprises a groove.

In one preferred embodiment, there is provided an ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a first pursing strip of the one or more pursing strips comprises two side edges corresponding to the two lateral edges of the drain and a line of weakness spanning between the side edges, and the line of weakness is curved, and the one or more pursing strips comprises a narrow pursing strip that has a smaller width than the first pursing strip.

In one preferred embodiment, there is provided an ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a first pursing strip of the one or more pursing strips comprises two side edges corresponding to the two lateral edges of the drain and a line of weakness spanning between the side edges, and the line of weakness is curved and arced with a radius of curvature that is greater than the width of the pursing strip.

In one preferred embodiment, there is provided an ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a first pursing strip of the one or more pursing strips comprises two side edges corresponding to the two lateral edges of the drain and a line of weakness spanning between the side edges, and the line of weakness is curved and asymmetric about a line connecting the two side edges.

In one preferred embodiment, there is provided an ostomy appliance comprising a pouch and a drain, wherein the drain is formed of two walls attached to one another along two lateral edges of the drain to define a channel therebetween and the drain comprises a distal end for output of stomal effluent from the pouch and one or more pursing strips configured to separate the two walls of the drain when compressed in a direction perpendicular to the two lateral edges of the drain; wherein a first pursing strip of the one or more pursing strips comprises two side edges corresponding to the two lateral edges of the drain and a line of weakness spanning between the side edges, and the line of weakness is curved and convex on a side facing the distal end of the drain.

In one preferred embodiment, there is provided an ostomy appliance comprising a pouch and a drain, wherein the drain is formed of two walls attached to one another along two lateral edges of the drain to define a channel therebetween and the drain comprises a distal end for output of stomal effluent from the pouch and one or more pursing strips configured to separate the two walls of the drain when compressed in a direction perpendicular to the two lateral edges of the drain; wherein a first pursing strip of the one or more pursing strips comprises two side edges corresponding to the two lateral edges of the drain and a line of weakness spanning between the side edges, and the line of weakness is curved to resist folding of the pursing strip by at least 150 degrees about the line of weakness, and preferably to resist folding of the pursing strip by at least 90 degrees about the line of weakness.

In one preferred embodiment, there is provided an ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a first pursing strip of the one or more pursing strips comprises two side edges corresponding to the two lateral edges of the drain and a line of weakness spanning between the side edges, and the line of weakness is curved, wherein the drain is foldable about one or more fold lines into a closed configuration in which passage of stomal output through the drain is inhibited, and the line of weakness is provided on one side of at least one of the one or more fold lines.

In one preferred embodiment, there is provided an ostomy appliance comprising a pouch and a drain, the drain formed of proximal and distal walls each comprising two lateral edges, the drain comprising a distal end for output of stomal effluent from the pouch, wherein each lateral edge of one wall is attached to the corresponding lateral edge of the other wall to define a channel therebetween, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip, wherein the lines of weakness are arranged at an angle of at least 30 degrees from one another.

In one preferred embodiment, there is provided an ostomy appliance comprising a pouch and a drain, the drain formed of proximal and distal walls each comprising two lateral edges, the drain comprising a distal end for output of stomal effluent from the pouch, wherein each lateral edge of one wall is attached to the corresponding lateral edge of the other wall to define a channel therebetween, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip, wherein the lines of weakness are arranged to diverge in a direction towards the distal end of the drain.

In one preferred embodiment, there is provided an ostomy appliance comprising a pouch and a drain, the drain formed of proximal and distal walls each comprising two lateral edges, the drain comprising a distal end for output of stomal effluent from the pouch, wherein each lateral edge of one wall is attached to the corresponding lateral edge of the other wall to define a channel therebetween, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip, wherein only the first pursing strip comprises one or more lines of weakness.

In one preferred embodiment, there is provided an ostomy appliance comprising a pouch and a drain, the drain formed of proximal and distal walls each comprising two lateral edges, the drain comprising a distal end for output of stomal effluent from the pouch, wherein each lateral edge of one wall is attached to the corresponding lateral edge of the other wall to define a channel therebetween, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip, the two or more pursing strips further comprising a notched pursing strip, the notched pursing strip comprising a notch in a transverse edge of the notched pursing strip, the transverse edge being substantially parallel to the lateral edges of the drain.

In one preferred embodiment, there is provided an ostomy appliance comprising a pouch and a drain, the drain formed of proximal and distal walls each comprising two lateral edges, the drain comprising a distal end for output of stomal effluent from the pouch, wherein each lateral edge of one wall is attached to the corresponding lateral edge of the other wall to define a channel therebetween, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip, the two or more pursing strips further comprising a narrow pursing strip that has a smaller width than the first pursing strip.

In one preferred embodiment, there is provided an ostomy appliance comprising a pouch and a drain, the drain formed of proximal and distal walls each comprising two lateral edges, the drain comprising a distal end for output of stomal effluent from the pouch, wherein each lateral edge of one wall is attached to the corresponding lateral edge of the other wall to define a channel therebetween, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip, and the lines of weakness are each arranged at an angle by at least 15 degrees from a lateral edge of the drain.

According to a second aspect of the present invention there is provided a method of manufacturing an ostomy appliance comprising providing a pouch and two walls, forming a drain of the pouch by attaching the two walls to one another along two lateral edges of the drain to define a channel therebetween, and providing one or more pursing strips on the drain, wherein the one or more pursing strips are configured to separate the walls of the drain when compressed in a direction perpendicular to the two lateral edges of the drain, and a pursing strip of the one or more pursing strips comprises a transverse edge substantially perpendicular to the two lateral edges of the drain and the transverse edge comprises a notch spanning at least half of the transverse edge.

The method of the second aspect of the invention may be a method of manufacturing the ostomy appliance of the first aspect of the invention, which, of course, may include any optional feature outlined above.

The method may comprise forming the notch in the pursing strip through any suitable means, for example cutting or punching the notch out of the pursing strip. The method may comprise forming a notch in the pursing strip during formation of the pursing strip, for example curing the pursing strip in a mould that is shaped to result in a notch in the cured pursing strip.

The method may comprise moving the drain from the open configuration to the closed configuration. The method may comprise folding the drain about the pursing strips, such as the first and/or second pursing strip(s). The method may comprise retaining the drain in the closed configuration using the closure mechanism. Thus, the ostomy appliance is supplied to the user in a closed state and ready to be used immediately.

The method may comprise forming a line of weakness in the pursing strip through any suitable means, for example scoring, perforation, or forming (e.g. by pressing or cutting) a groove in the pursing strip. The method may comprise forming a line of weakness in the pursing strip during formation of the pursing strip, for example curing the pursing strip in a mould that is shaped to result in a line of weakness (such as a groove) in the cured pursing strip.

The method may comprise attaching the pursing strip to one of the two walls of the drain. The method may comprise attaching the pursing strip adjacent the distal end of the drain. The method may comprise arranging the pursing strip with the line of weakness on a distal side of the pursing strip from the distal end of the drain.

According to another aspect of the present invention there is provided a method of manufacturing an ostomy appliance comprising providing a pouch and proximal and distal walls, forming a drain of the pouch by attaching the proximal and distal walls to one another along two lateral edges of the drain to define a channel therebetween, and providing two or more pursing strips on the drain, wherein the drain comprises a distal end for output of stomal effluent from the pouch, the two or more pursing strips are configured to separate the walls of the drain when compressed in a direction perpendicular to the two lateral edges of the drain, and each of the two or more pursing strips comprises an upper edge and a lower edge both perpendicular to the lateral edges of the drain, wherein the method comprises providing a first pursing strip, forming two converging lines of weakness in the first pursing strip that span between the upper and lower edges, arranging the first pursing strip on the proximal wall at the distal end of the drain and providing a second pursing strip arranged on the distal wall wherein, a longitudinal gap is provided between the upper edge of the first pursing strip and a lower edge of the second pursing strip.

According to another aspect of the present invention there is provided a method of manufacturing an ostomy appliance comprising providing a pouch and two walls, forming a drain of the pouch by attaching the two walls to one another along two lateral edges of the drain to define a channel therebetween, and providing one or more pursing strips on the drain, wherein the one or more pursing strips are configured to separate the walls of the drain when compressed in a direction perpendicular to the two lateral edges of the drain, the method comprising forming a line of weakness in a pursing strip of the one or more pursing strips between two side edges of the pursing strip, wherein the two side edges of the pursing strip correspond to the lateral edges of the drain and the line of weakness is curved.

According to a third aspect of the present invention there is provided a method of draining an ostomy appliance comprising a pouch and a drain, the drain comprising two walls sealed together along two lateral edges of the drain to define a channel therebetween, the method comprising compressing a pursing strip of the drain in a direction perpendicular to the two lateral edges of the drain to separate the two walls, wherein the pursing strip comprises a transverse edge substantially perpendicular to the two lateral edges of the drain and the transverse edge comprises a notch spanning at least half of the transverse edge.

The method of the third aspect of the invention may be a method of draining the ostomy appliance of the first aspect of the invention, which, of course, may include any optional feature outlined above and may be manufactured according to the second aspect of the invention.

The method may comprise moving the drain from the closed configuration to the open configuration. The method may comprise moving drain from closed configuration to the open configuration by unrolling and/or unfolding the drain.

The method may comprise releasing the one or more fastening elements.

The method may comprise releasing the pursing strip and allowing it to flatten. The method may comprise moving the drain from the open configuration to the closed configuration after draining. The method may comprise folding the drain about the pursing strip. The method may comprise retaining the drain in the closed configuration using the closure mechanism. Thus, the ostomy appliance is re-sealed so that it can be re-used by the user.

According to another aspect of the present invention there is provided a method of draining an ostomy appliance comprising a pouch and a drain, the drain comprising proximal and distal walls sealed together along two lateral edges of the drain to define a channel therebetween, wherein the drain comprises two or more pursing strips each comprising an upper edge and a lower edge both of which are perpendicular to the lateral edges of the drain, the method comprising compressing a first pursing strip of the two or more pursing strips in a direction perpendicular to the two lateral edges of the drain to separate the two walls, wherein the first pursing strip comprises two converging lines of weakness spanning between the upper and lower edges of the first pursing strip, the first pursing strip is arranged on the proximal wall at the distal end of the drain, a second pursing strip is arranged on the distal wall and a longitudinal gap is provided between the upper edge of the first pursing strip and a lower edge of the second pursing strip.

According to another aspect of the present invention there is provided a method of draining an ostomy appliance comprising a pouch and a drain, the drain comprising two walls sealed together along two lateral edges of the drain to define a channel therebetween, the method comprising compressing a pursing strip of the drain in a direction perpendicular to the two lateral edges of the drain to separate the two walls, wherein the pursing strip comprises a line of weakness spanning between two side edges of the pursing strip that correspond to the two lateral edges of the drain, wherein the line of weakness is curved.

The method may comprise changing the position of the line of weakness with respect to a lower edge of the pursing strip less than the position of the line of weakness with respect an upper edge of the pursing strip. The lower edge may be arranged at the distal end of the drain. The upper edge may be at the other end of the pursing strip from the lower edge. The upper edge and lower edge may span all the way between the side edges of the pursing strip. The method may comprise changing the curvature of the pursing strip between the side edges more at points between the line of weakness and the lower edge than at points between the line of weakness and the upper edge.

According to a fourth aspect of the present invention there is provided a method of using an ostomy appliance according to the first aspect of the present invention, the method comprising attaching the pouch to the body of the user around a stoma and allowing stomal effluent to pass from the stoma and into the pouch.

The method of the fourth aspect of the invention may be a method of using an ostomy appliance manufactured according to the second aspect of the invention.

The method of the fourth aspect may include draining the ostomy appliance according to the method of the third aspect.

The methods of the second to fourth aspects, including any methods described generally herein, may of course include any one or more features of one another as well as the features of the ostomy appliance of the first aspect or an ostomy appliance, ostomy drain or pursing strip as described generally herein.

### Detailed Description of the Invention

In order that the invention may be more clearly understood one or more embodiments thereof will now be described, by way of example only, with reference to the accompanying drawings, of which:
- Figure 1: is a schematic front view of a first embodiment of an ostomy appliance with the drain in an open configuration;
- Figure 2: is a schematic rear view of the ostomy appliance of Figure 1;
- Figure 3: is a schematic plan view of a pursing strip of the ostomy appliance of Figure 1;
- Figure 4: is a schematic cross-section along line A-A of Figure 1 with the first pursing strip not compressed;
- Figure 5: is a schematic view along line B-B of Figure 2 with the first pursing strip not compressed;
- Figure 6: is a schematic cross-section along line A-A of Figure 1 with the first pursing strip compressed;
- Figure 7: is a schematic view along line B-B of Figure 2 with the first pursing strip compressed;
- Figure 8: is a schematic front view of the ostomy appliance of Figure 1 with the drain in a closed configuration;
- Figure 9: is a schematic front view of a second embodiment of an ostomy appliance with the drain in an open configuration;
- Figure 10: is a schematic rear view of the ostomy appliance of Figure 9;
- Figure 11: is a schematic plan view of a third pursing strip of the ostomy appliance of Figure 9;
- Figure 12: is a schematic cross-section along line A-A of Figure 9 with the first and third pursing strip not compressed;
- Figure 13: is a schematic view along line B-B of Figure 10 with the first and third pursing strip not compressed;
- Figure 14: is a schematic cross-section along line A-A of Figure 9 with the first and third pursing strip compressed;
- Figure 15: is a schematic view along line B-B of Figure 10 with the first and third pursing strip compressed;
- Figure 16: is a schematic front view of a third embodiment of an ostomy appliance with the drain in an open configuration;
- Figure 17: is a schematic rear view of the ostomy appliance of Figure 16;
- Figure 18: is a schematic plan view of a third pursing strip of the ostomy appliance of Figure 16;
- Figure 19: is a schematic cross-section along line A-A of Figure 16 with the first and third pursing strip not compressed;
- Figure 20: is a schematic view along line B-B of Figure 17 with the first and third pursing strip not compressed;
- Figure 21: is a schematic cross-section along line A-A of Figure 16 with the first and third pursing strip compressed;
- Figure 22: is a schematic view along line B-B of Figure 17 with the first and third pursing strip compressed;
- Figure 23: is a schematic cross-sectional side-view of the drain of a fourth embodiment of an ostomy appliance with the drain not compressed;
- Figure 24: is a schematic end-view of the drain of Figure 23;
- Figure 25: is a schematic end-view of the drain of Figure 23 with the first pursing strip compressed and the second pursing strip not compressed;
- Figure 26: is a schematic end-view of the drain of Figure 23 with the first and second pursing strips compressed;
- Figure 27: is a schematic front view of a fifth embodiment of an ostomy appliance with the drain in an open configuration;
- Figure 28: is a schematic rear view of the ostomy appliance of Figure 27;
- Figure 29: is a schematic plan view of a pursing strip of the ostomy appliance of Figure 27;
- Figure 30: is a schematic cross-section along line A-A of Figure 27 with the first pursing strip not compressed;
- Figure 31: is a schematic view along line B-B of Figure 28 with the first pursing strip not compressed;
- Figure 32: is a schematic cross-section along line A-A of Figure 27 with the first pursing strip compressed;
- Figure 33: is a schematic view along line B-B of Figure 28 with the first pursing strip compressed;
- Figure 34: is a schematic front view of the ostomy appliance of Figure 27 with the drain in a closed configuration;
- Figure 35: is a schematic front view of a sixth embodiment of an ostomy appliance with the drain in an open configuration;
- Figure 36: is a schematic rear view of the ostomy appliance of Figure 35;
- Figure 37: is a schematic plan view of a pursing strip of the ostomy appliance of Figure 35;
- Figure 38: is a schematic cross-section along line A-A of Figure 35 with the first pursing strip not compressed;
- Figure 39: is a schematic view along line B-B of Figure 36 with the first pursing strip not compressed;
- Figure 40: is a schematic cross-section along line A-A of Figure 35 with the first pursing strip compressed;
- Figure 41: is a schematic view along line B-B of Figure 36 with the first pursing strip compressed;
- Figure 42: is a schematic front view of the ostomy appliance of Figure 35 with the drain in a closed configuration;
- Figure 43: is a schematic cross-sectional side-view of the drain of a seventh embodiment of an ostomy appliance with the drain not compressed;
- Figure 44: is a schematic end-view of the drain of Figure 43;
- Figure 45: is a schematic end-view of the drain of Figure 43 with the first and second pursing strips compressed and the third pursing strip not compressed;
- Figure 46: is a schematic end-view of the drain of Figure 43 with the first, second and third pursing strips are compressed;
- Figure 47: is a schematic cross-sectional side-view of the drain of an eighth embodiment of an ostomy appliance with the drain not compressed;
- Figure 48: is a schematic end-view of the drain of Figure 47;
- Figure 49: is a schematic end-view of the drain of Figure 47 with the first and second pursing strips compressed and the third pursing strip not compressed; and
- Figure 50: is a schematic end-view of the drain of Figure 47 with the first, second and third pursing strips are compressed.

As is conventional, in the Figures broken lines are used to denote hidden features.

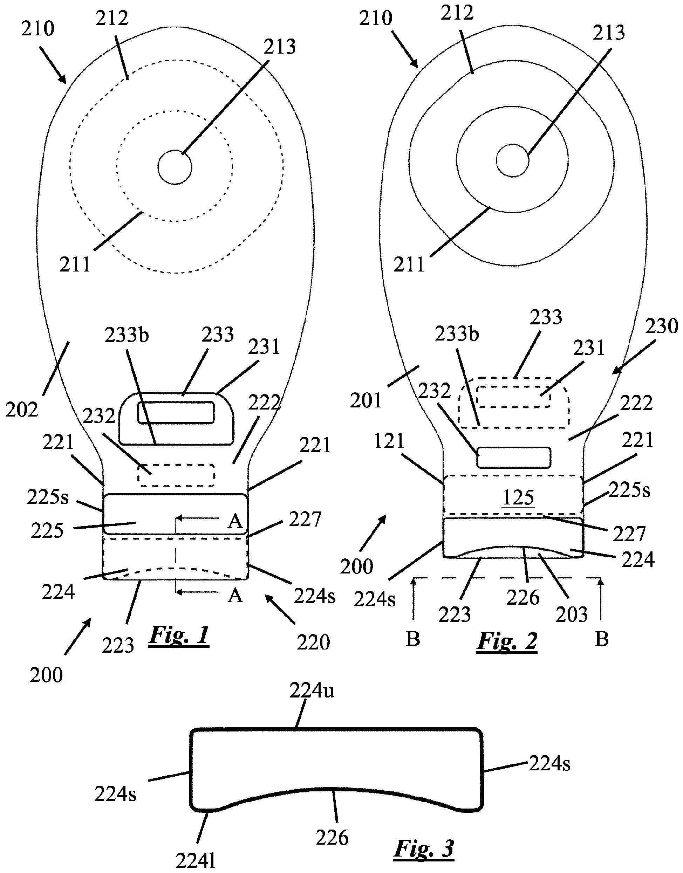

Referring to Figures 1-8, a first embodiment of an ostomy appliance 200 comprises a pouch 210, a drain 220 and a closure mechanism 230. The pouch 210 and drain 220 are both formed from two walls of material sealed about their periphery. Consequently, in this embodiment the pouch 210 and drain 220 are integrally formed. The two walls comprise a proximal wall 201 intended to be closest to the body of the user in use and a distal wall 202 on an opposite side of the ostomy appliance 200 to the proximal wall 201. In this embodiment, the two walls 201, 202 are both formed of a flexible plastics material such as ethylene-vinyl acetate (EVA).

In this embodiment, the drain 220 is arranged at a bottom of the ostomy appliance 200 in use such that stomal output in the pouch 210 is able to flow down towards and through the drain 220 under the action of gravity.

In this embodiment, the pouch 210 comprises a stomal inlet 211 arranged towards a top of the ostomy appliance 200 distal from the drain 220 and on the proximal wall 201. The stomal inlet 211 comprises a circular aperture in the proximal wall 201 to surround the user's stoma in use and allow stomal output to pass from the stoma and into the pouch 210.

In this embodiment, the pouch 210 is "one piece" and comprises an adhesive collar 212 arranged around the stomal inlet 211. The adhesive collar 212 is annular with an inner perimeter defined by the stomal inlet 211 and an outer perimeter that spans about twice the width of the inner perimeter but is completely within the outer edges of the ostomy appliance 200. In this embodiment, the outer perimeter has the square-diamond shape with rounded edges, of course in other embodiments different sizes/shapes may be used. The adhesive collar 212 is formed from a mouldable adhesive material such as a hydrocolloid adhesive. The adhesive collar 212 is attached to the proximal wall 201 and is used to attach the ostomy appliance 200 to the body of a user around the stoma.

In this embodiment, the adhesive collar 212 is provided with a release liner (not shown) to protect the adhesive collar 212 before use and ensure that it does not inadvertently adhere to other objects before use.

In an alternative embodiments, the adhesive collar may be replaced with a coupling element to fit onto a corresponding coupling element that is provided with an adhesive collar. In such embodiments, the ostomy appliance may be a two-piece ostomy appliance.

In this embodiment, the pouch 210 comprises an optional gas vent 213 provided in the distal wall 202 and in register with the stomal inlet 211. The gas vent 213 is configured to allow gases in the pouch 210 to leave the pouch 210 and in this embodiment comprises a deodorising material to neutralise and/or mask any unpleasant smells associated with the gases leaving the pouch 210.

In this embodiment, the drain 220 extends from the pouch 210 at the bottom of the ostomy appliance 200. The drain 220 is formed by a seal between the walls 201, 202 along the two lateral edges 221 of the drain 220, thus forming a channel therebetween. In this embodiment, the drain 220 is rectangular with a width defined between the lateral edges 221 of the drain 220 and a length perpendicular to its width. The length of the drain 220 is about 25-35% larger than the width of the drain 220, for example about 30% larger. In this embodiment, the width of the drain 220 is about 70 mm.

In this embodiment, the drain 220 comprises a proximal end 222 to receive stomal output from the pouch 210 and which corresponds to the bottom of the pouch referred to herein, and an opposite distal end 223 to allow stomal output to exit the drain 220 and ostomy appliance 200. The length of the drain is thus measured between the proximal and distal ends 222, 223.

In this embodiment, the drain 220 comprises a first pursing strip 224 and a second pursing strip 225. The first and second pursing strips 224, 225 are both the same rectangular shape with a width spanning the entire width of the drain 220, e.g. about 70 mm, and a length approximately one third to one quarter their width, for example about 20 mm. The first and second pursing strips 224, 225 are both flat in that (at rest) they have an intrinsic curvature of zero; this helps keep the drain 220 and ostomy appliance 200 compact.

The first and second pursing strips 224, 225 are formed of a material that is more rigid than the walls 201, 202 that form the drain 220, for example high density polyethylene (HDPE) or polystyrene.

In this embodiment, both the first and second pursing strips 224, 225 comprise a pair of side edges 224s, 225s that are aligned with and positioned over the lateral edges 221 of the drain 222. The first and second pursing strips 224, 225 also each comprise an upper edge 224u, 225u and a lower edge 224l, 225l, the lower edges 224l, 225l being closer to the distal end 223 of the drain 220 than the upper edges 224u, 225u. The first pursing strip 224 is arranged on the proximal wall 201 at the distal end 223 of the drain 220 with the lower edge 224l of the first pursing strip 224 aligned with and positioned over the distal end 223 of the drain 220. The second pursing strip 225 is arranged on the distal wall 202 at a distance from the distal end 223 of the drain 220 that is greater than the length of the first pursing strip 224 such that a thin lateral gap 227 is present between the two pursing strips 224, 225. In this embodiment, the first and second pursing strips 224, 225 thus define a fold line of the drain 220 at the position of the lateral gap 227 due to higher rigidity of the pursing strips 224, 225.

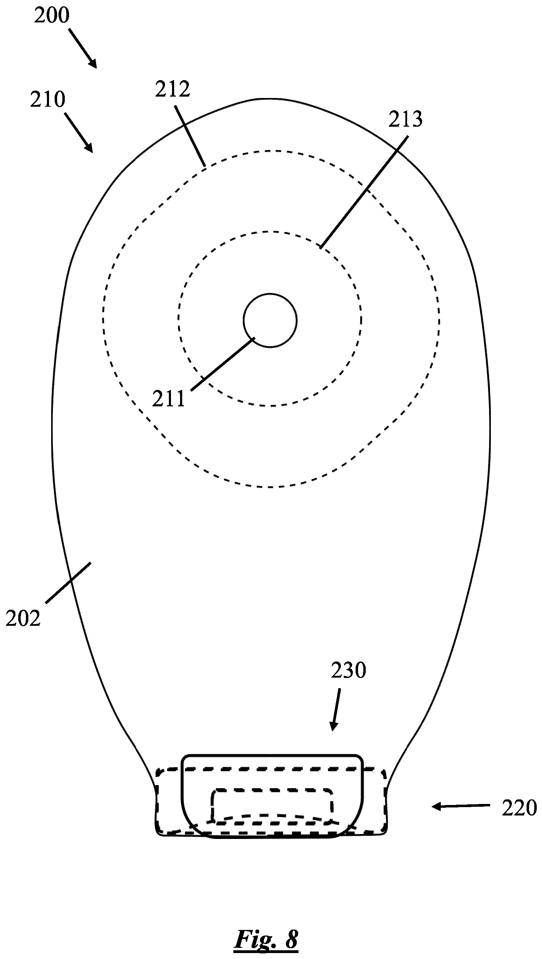

In this embodiment, the drain 220 comprises an open configuration in which passage of stomal output through the drain 220 is permitted. In the open configuration, the drain 220 is unfolded to define a substantially linear channel therethrough, for example as shown in Figures 1 and 2. In this embodiment, the drain 220 comprises a closed configuration in which passage of stomal output through the drain 220 is inhibited. In the closed configuration, the drain 220 is folded to define a closed tortuous channel, for example as shown in Figure 8.

Referring to Figures 3-7, in this embodiment the first pursing strip 224 is configured to separate the walls 201, 202 of the drain 220 when compressed in a direction perpendicular to the lateral edges 221 of the drain 220. In order to improve the ability of the first pursing strip 224 to separate the walls 201, 202 of the drain 220, the first pursing strip 224 comprises a notch 226 in its lower edge 224l. This can help to encourage the walls 201, 202 to separate even when wet. The lower edge 224l may also be referred to as a transverse edge as it is substantially perpendicular to the lateral edges of the drain.

In this embodiment, as the first pursing strip 225 is arranged on the proximal wall 201 with the lower edge 224l and notch 226 aligned with and positioned over the distal end 223 of the drain 220. This configuration allows the first pursing strips 224 to open the drain 420 when compressed as described further below.

In this embodiment, the notch 226 spans about 85% of the lower edge 224l and is positioned centrally in the lower edge 224l. The notch 226 is symmetrical about the midline of the pursing strip, which corresponds to a longitudinal axis of symmetry of the pouch 210. This helps to ensure more even and predictable deformation during opening of the drain 223 while also providing a significant difference between the mechanical properties of the upper and lower edges 224u, 224l of the first pursing strip 224 to help in separating the proximal and distal walls 201, 202.

In this embodiment, the notch 226 has a width measured parallel to the lower edge 224l and a length perpendicular to its width. The notch 226 has an aspect ratio (width:length) of about 12:1, this helps to ensure the third pursing strip 226 retains sufficient strength and flexibility to prevent damage during bending and opening of the drain 223.

In this embodiment, the notch 226 is arced. The notch 226 has a radius of curvature that is about 50% greater than the width of the first pursing strip 224 as measured between the side edges 224s of the first pursing strip. This shape ensures the first pursing strip 224 deforms more predictably which helps to improve opening performance.

In this embodiment, the distal end 223 of the drain 220 is straight. Consequently, an end region 203 of the proximal wall 201 is exposed where the notch 226 cuts into the first pursing strip 224. The end region 203 can therefore flex and move away from the first pursing strip 224 as it is not attached to any of the pursing strips directly. This further increases the propensity that the proximal and distal walls 201, 202 will separate during opening.

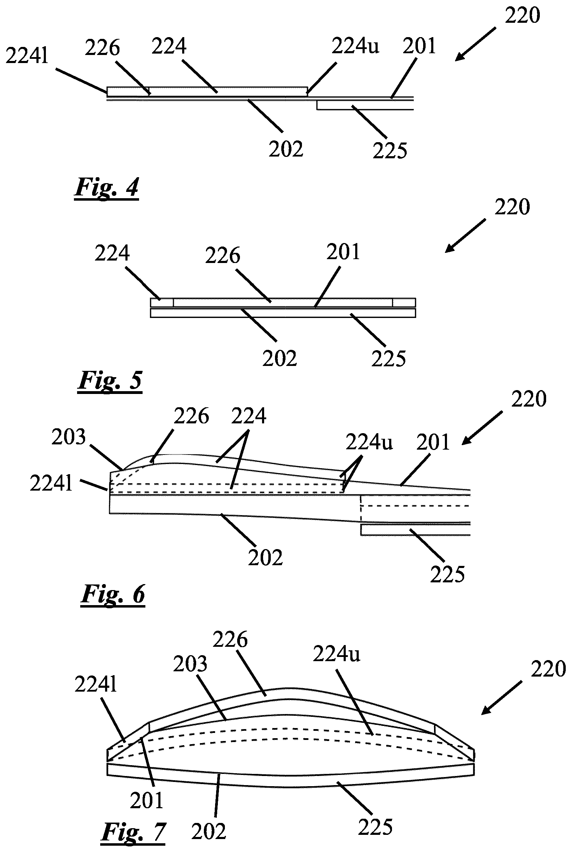

Referring to Figures 4-7, in this embodiment the first pursing strip 224 is compressed to open the drain 220 and separate the proximal and distal walls 201, 202. When compressed, the notch 226 provides an imbalance in the mechanical properties of the first pursing strip 224 that causes the proximal and distal walls 201, 202 to separate. The lower edge 224l of the third pursing strip 224 is weaker than the upper edge 224u and therefore flexes more under compression which results in a larger displacement away from the normally closed position at the lower edge 224l and opening of the drain 220.

In this embodiment, when uncompressed, the notch 226 allows the first pursing strip 224 to return to a flat configuration which facilitates more efficient closure and storage of the drain as described below.

In this embodiment, the closure mechanism 230 is configured to retain the drain 220 in the closed configuration and the exemplary closure mechanism comprises two fastening elements: a pouch fastening element 231 and a drain fastening element 232. The fastening elements 231, 232 are the same shape and size; both are rectangular with lengths and widths approximately half those of the first and second pursing strips 224, 225. The fastening elements 231, 232 comprise complementary fastening surfaces in the form of mushroom-head fasteners configured to engage one another when pressed into contact, of course in other embodiments different fastening surfaces such as hook and hook or hook and loop fasteners may be used.

In this embodiment, the drain fastening element 232 is positioned on the proximal wall 201 and between the proximal end 222 of the drain 220 and the second pursing strip 225. In this embodiment, the pouch fastening element 231 is positioned on a closure flap 233 that is attached to the distal wall 202. The closure flap 233 is attached to the distal wall 202 along a base 233b of the closure flap 233. The closure flap 233 is formed of a flexible plastics material, such as that used to form the walls 201, 202 and consequently, the closure flap 233 can bend to pivot with respect to the pouch 210 about the base 233b. The closure mechanism 230 and drain 220 are arranged such that when the drain 220 is in the closed configuration, the drain 220 is folded up between the distal wall 202 and under the closure flap 233 such that the closure flap 233 can be rotated about its base 233b until the pouch fastening element 231 contacts the drain fastening element 232, for example as described below in more detail.

In this embodiment, in order to manufacture the ostomy appliance 200, the two walls 201, 202 may be provided pre-cut into the required shape. The stomal inlet 211 may be formed in the proximal wall 201, or may be pre-cut in a different embodiment. The pouch fastening element 231 can then be attached to the closure flap 233, for example using adhesive, and then the closure flap 233 may be attached to the distal wall 202 by sealing the base 233b to the wall 202, for example by welding. The drain fastening element 232 may then be added to the proximal wall 201.

In this embodiment, the adhesive collar 212 may then be attached to the proximal wall 201 and a release liner placed over the adhesive collar 212. The gas vent 213 may be formed in the distal wall 202, for example by making a small hole in the distal wall 202 and fitting a deodorising filter over the hole.

In this embodiment, the two walls 201, 202 can then be sealed together around their periphery except at the distal end 223 of the drain 220. The first pursing strip 224 is then provided either with the notch 226 pre-formed, or the notch 226 can then be formed for example by cutting the first pursing strip 224. The second pursing strip 225 is then provided and the first and second pursing strips 224, 225 are attached to the proximal and distal walls 201, 202 respectively in the positions described above.

In this embodiment, before being provided to the user, the drain 220 is moved into the closed configuration by folding the drain 220 along a line between the first and second pursing strips 224, 225 (e.g. at the lateral gap 227) with the distal wall 202 on the inside of the fold such that the first pursing strip 224 overlies the second pursing strip 225. The drain 220 is then folded again twice more in the same manner so as to effectively roll up the distal end 223 of the drain 220 within the folded drain 220. The final fold of the drain 220 is about the proximal end 222 of the drain 220 and consequently the entire drain 220 overlaps the distal wall 202 of the pouch 210 in the closed configuration.

In this embodiment, as the drain fastening element 232 is positioned on the proximal wall 201, when the drain 220 is folded the drain fastening element 232 pivots around to a position where it is exposed and facing away from the distal wall 202 of the pouch 210. In this position, the closure flap 233 may be pivoted over the drain 220 and the pouch fastening element 231 brought into contact with the drain fastening element 232 to seal the drain 220 in the closed configuration. The ostomy appliance 200 is now ready to be supplied to a user.

In this embodiment, to use the ostomy appliance 200, the user first removes the optional release liner and then attaches the ostomy appliance 200 to the body with the user's stoma surrounded by the stomal inlet 211. This allows stomal output to pass from the body and into the pouch 210 where it collects due to the drain 220 being provided in the closed configuration.

In this embodiment, the user may empty the contents of the pouch 210, for example when it is full or otherwise necessary to do so, by moving the drain 220 into the open configuration. To do this, the user grasps the closure flap 233 in one hand and the pouch 220 in the other hand and moves the closure flap 233 and pouch 220 apart to release the pouch fastening element 231 from the drain fastening element 232. The drain 220 can then by unfolded/unrolled completely such that it defines a substantially linear channel from the proximal end 222 to the distal end 223 of the drain 220.

Referring to Figures 4-7, in this embodiment, the walls 201, 202 of the drain 220 can be separated by compressing the first pursing strip 224 in a direction perpendicular to the side edges 224s of the first pursing strip 224 or parallel to the width of the drain 220; for example, the user may pinch side edges 224s together between their fingers and thumb. This causes the first pursing strip 224 to bend out of the plane of the drain 220.

When compressed in this manner the first pursing strip 224 flexes in a non-uniform manner to separate the two walls 201, 202. This is guided by the notch 226 which generates an imbalance in the mechanical properties of the first pursing strip 224 both along its length and along its width.

In this embodiment, as the proximal wall 201 is attached to the first pursing strip 224 across the entire area of the first pursing strip 224, the proximal wall 201 follows the shape of the first pursing strip 224 and this leads it to separate from the distal wall 202 more easily.

In this embodiment, now that the walls 201, 202 are separated, the ostomy appliance 200 can be emptied by allowing the contents of the pouch 210 to flow out of the drain 220. After emptying, the drain 220 can be resealed by moving the drain 220 from the open configuration to the closed configuration and sealing it closed using the closure mechanism 230 as described above. The ostomy appliance 200 can then be refilled, emptied and resealed repeatedly by the user.

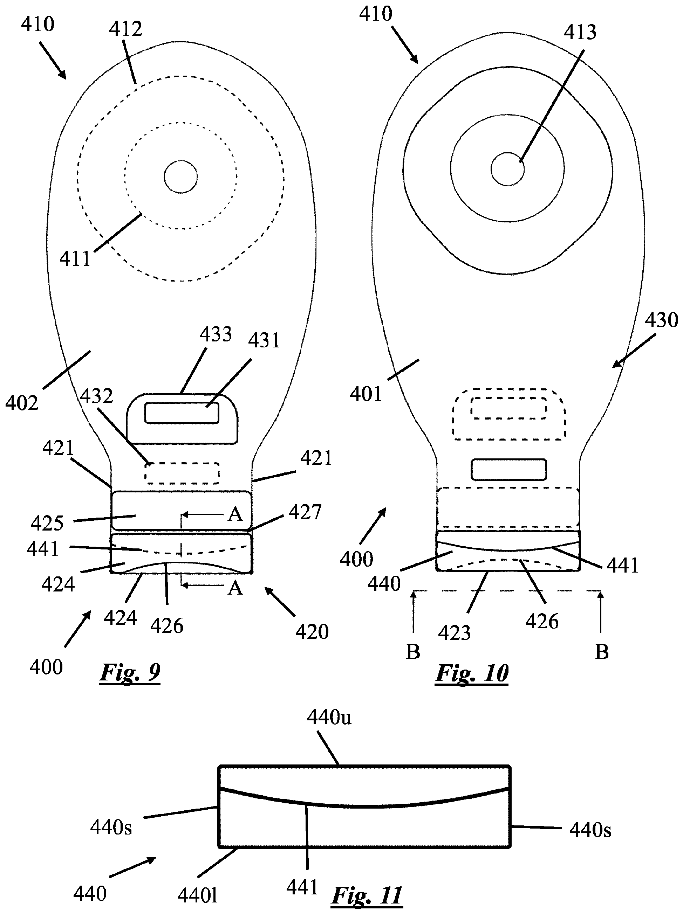

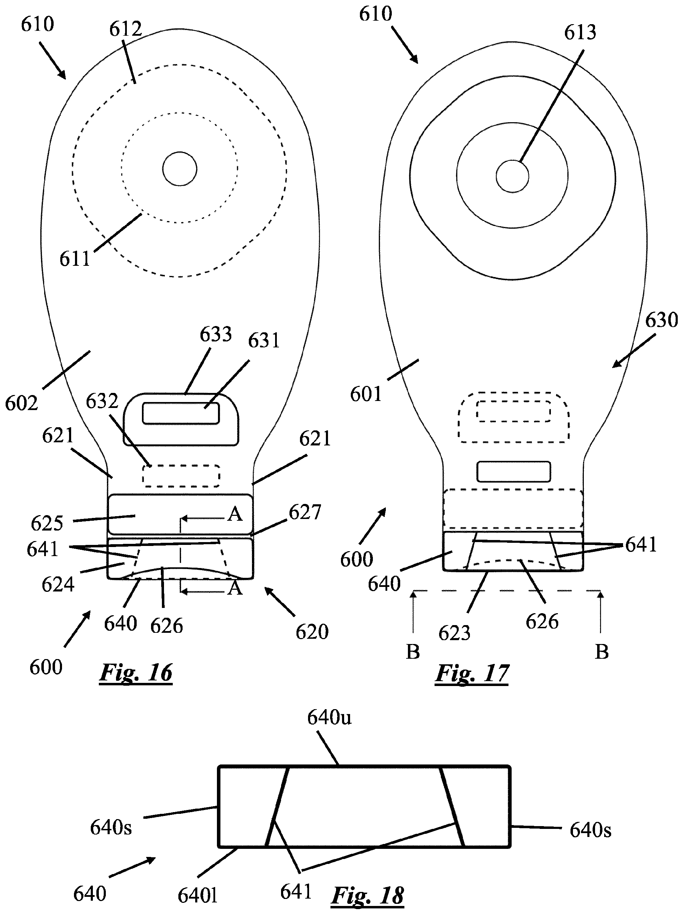

A second embodiment of an ostomy appliance 400 is shown Figures 9 - 15 and a third embodiment of an ostomy appliance 600 is shown in Figures 16 - 22. The second and third embodiments share many of the same features of the first embodiment above and consequently only the differences are described below and like numerals are used to denote like features. The main difference between the first embodiment and the second and third embodiments is that in the second and third embodiments the first pursing strip 424, 624 is attached to the distal wall 402, 602 in the same manner as it is attached to the proximal wall 201 in the first embodiment. Consequently, it exposes an end region 404, 604 of the distal wall 402, 602 rather than the proximal wall as in the first embodiment. However, otherwise, the first pursing strip 424, 624 is the same as that described in the first embodiment and has all the same features.

In the second and third embodiments, the ostomy appliance 400, 600 comprises three pursing strips: a first pursing strip 424, 624, a second pursing strip 425, 625 and a third pursing strip 440, 640. The second pursing strip 425, 625 has all the same features as the second pursing strips of the first embodiment described above. The third pursing strip 440, 640 has the same properties as the second pursing strip 425, 625 except that comprises at least one line of weakness 441, 641. The at least one line of weakness provides the third pursing strip 440, 640 with mechanical properties that allow it to work with the first pursing strip 424, 624 to more easily separate the walls of the drain 420, 620 as described further below. The third pursing strip 440, 640 may therefore be referred to as a weakened pursing strip.

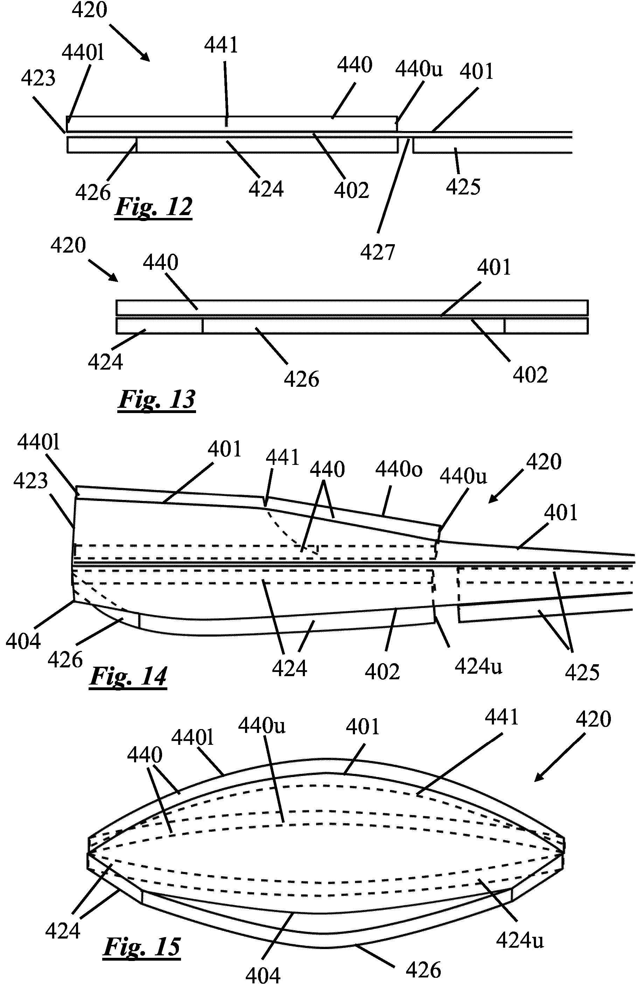

In the second and third embodiments, as best seen in Figures 12 and 14, and 20 and 22 respectively, each line of weakness 441, 641 comprises a groove in an outside surface 440o, 640o of the third pursing strip 440, 640 distal from the walls of the drain 420, 640. The lines of weakness 441, 641 are therefore scored or cut out of the third pursing strip 440, 640 to allow it to bend more easily about the line of weakness 441, 641 as described below. In this embodiment, each groove has a width perpendicular to each line of weakness of about 0.25mm.

In the second and third embodiments, the third pursing strip 440, 640 is positioned on the proximal wall 401, 601 at the same place that the first pursing strip is positioned in the first embodiment described above. In the second and third embodiments, the first and third pursing strips are therefore on either side of the distal end of the drain 420, 620.

In the second and third embodiments, when closing and opening the drain 420, 620, the third pursing strip 440, 640 provides an additional rigid element to help fold the drain 420, 640 around. This can improve the sealing action of the drain as it can be folded more tightly.

Referring to Figures 9 - 15, in the second embodiment, the at least one line of weakness comprises a line of weakness 441 spanning completely between the side edges 440s of the third pursing strip 440 (all the way from one side edge 440s to the opposing side edge 440s). Consequently, the line of weakness 441 is a transverse line of weakness. The line of weakness 441 is curved such that it is asymmetrical about lines connecting the two side edges 440s and lines perpendicular to the side edges 440s. In addition, the line of weakness 441 is convex on a side facing the distal end of the drain and thus the curvature of the line of weakness 441 and notch 426 is opposing. In addition the notch 426 and line of weakness 441 are not overlapping, so they each act on different parts of the drain. Consequently, when compressed, the first and third pursing strips 424, 440 flex in a non-uniform manner that tends to cause the walls 401, 402 to separate even when stuck to each other, such as when wet.

In this embodiment, the line of weakness 441 is arranged in a half of the third pursing strip 440 distal from the distal end 423 of the drain 420. The line of weakness 441 meets the side edges 440s at approximately three-quarters of the length of the third pursing strip 440 from its lower edge 440l, which corresponds to the points on the line of weakness 441 furthest from the lower edge 440l. A midpoint of the line of weakness 441 is the point on the line of weakness 441 closest to the lower edge 440l and is arranged at a midpoint of the third pursing strip 440, both in terms of length and width. Consequently, the line of weakness 441 only spans about one quarter the length of the third pursing strip 440.

In this embodiment, the line of weakness 441 is an arc and is parallel to the lower edge 440l at the midpoint along the line of weakness 441. The line of weakness 441 curves away from the lower edge 440l either side of the midpoint and has a radius of curvature that is about twice the width of the third pursing strip 440, for example about 140 mm. The line of weakness 441 is therefore symmetric about a line extending midway between the side edges 440s of the third pursing strip 440.

Referring to Figures 12-15, in this embodiment, the walls 401, 402 of the drain 420 can be separated by compressing the first and third pursing strips 424, 440 in a direction perpendicular to the side edges 440s of the third pursing strip 440 or parallel to the width of the drain 420; for example, the user may pinch side edges 440s together between their fingers and thumb. This causes the third pursing strip 440 to bend out of the plane of the drain 420.

When compressed in this manner the first pursing strip 424 acts as described in relation to the first embodiment but on the distal wall 402, and the third pursing strip 440 also flexes in a non-uniform manner to separate the two walls 401, 402. This is guided by the line of weakness 441 which generates an imbalance in the mechanical properties of the third pursing strip 440 both along its length and along its width.

During compression the angle of the pursing strip between the line of weakness 441 and lower edge 440l changes less than the angle of the pursing strip between line of weakness 441 and the upper edge 440u (See Figures 12 and 14). However, simultaneously the curvature of the third pursing strip 440 between the side edges 440s changes more at points between the line of weakness 441 and the lower edge 440l than between the line of weakness 441 and the upper edge 440u (see Figures 13 and 15). Thus, the line of weakness 441 creates a situation where there are different changes in angle and curvature of the third pursing strip 440 either side of the line of weakness 441 to help separate the walls 401, 402 of the drain.

In this embodiment, as the proximal wall 401 is attached to the third pursing strip 440 across the entire area of the third pursing strip 440, the proximal wall 401 follows the shape of the third pursing strip 440 and this leads it to separate from the distal wall 402 more easily.

Of course, in this embodiment, the drain remains foldable about the fold lines defined by the edges of the pursing strips. The drain is not intended to be foldable about the line of weakness as it is a curved groove which cannot be easily folded.

Referring to Figures 16-22, in the third embodiment, the at least one line of weakness comprises two converging lines of weakness 641 spanning all the way from the upper edge 640u and lower edge 640l of the third pursing strip 640. Each of the lines of weakness 641 are therefore longitudinal lines of weakness. Each of the lines of weakness 641 are straight and angled such that they are asymmetrical about lines connecting the two side edges 640s and lines perpendicular to the side edges 640s. Consequently, when compressed, the third pursing strip 640 flexes in a non-uniform manner that tends to cause the walls 601, 602 to separate even when stuck to each other, such as when wet.

In this embodiment each of the lines of weakness 641 are angled with respect to each other by about 30 degrees, and as such are angled with respect to the side edges 640s (and the longitudinal axis of the appliance) by about 15 degrees. In addition, the lines of weakness 641 are mirror images of one another about the longitudinal axis of the drain (i.e. a line extending parallel to the side edges 640s and midway between the side edges 640s). This helps to ensure asymmetric bending along the length of the third pursing strip 640 which encourages the drain 620 to open as described below.

In this embodiment, the lines of weakness 641 are closer to one another along the upper edge 640u than the lower edge 640l of the third pursing strip 624. Nevertheless, along the upper edge 640u, the lines of weakness 641 are still separated by approximately half the width of the third pursing strip 640. Consequently, while the lines of weakness 641 are converging they do not converge which helps ensure the third pursing strip 640 can flex a significant distance out of the plane of the drain 620 and open the drain 620.

In this embodiment, each line of weakness 641 is arranged in a respective half of the third pursing strip 640. Each line of weakness 641meets the upper edge 640u at a point about one quarter from a respective side edge 640s across the upper edge 640u. Each line of weakness 641 meets the lower edge 640l at a point about 15% from a respective side edge 640s across the lower edge 640l. Consequently, each line of weakness 641 spans the entire length, and about 10% of the width of the third pursing strip 640.

In view of the above, in this embodiment the lines of weakness 641 diverge towards the distal end 623 of the drain 620. This is beneficial as it ensures the distal end of the drain opens widest which helps efficient passage of stomal output out of the drain. 620.

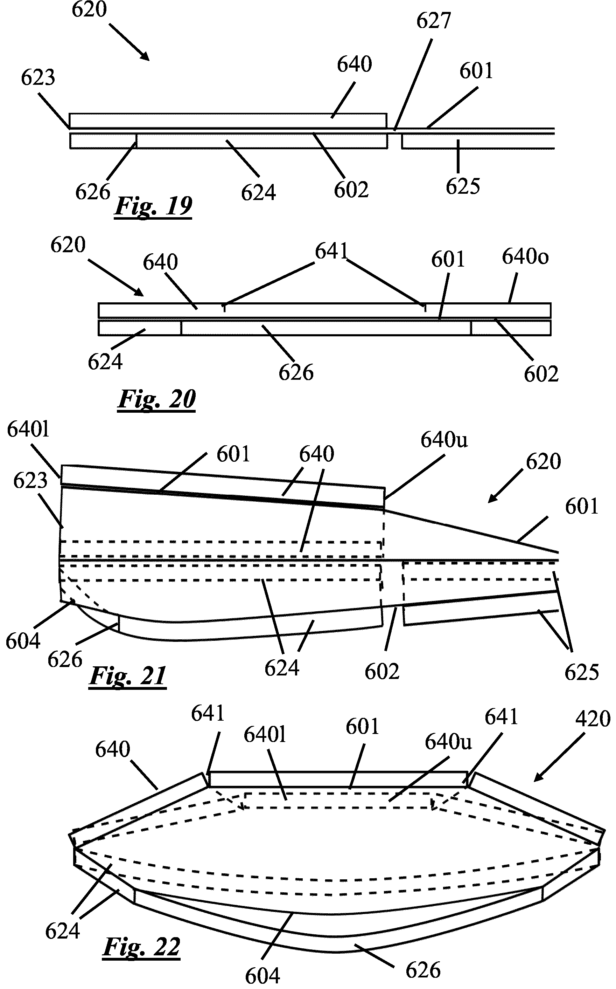

Referring to Figures 19-22, in this embodiment the first and third pursing strips 624, 640 are compressed to open the drain 620 and separate the proximal and distal walls 601, 602. When compressed the first pursing strip 624 acts as described in relation to the first embodiment but on the distal wall 602. **In** addition, during compression the angle of the third pursing strip 640 between each line of weakness 641 and side edges 640s changes less closer to the upper edge 640u than at points closer to the lower edge 640l, which creates a twisting of the third pursing strip between the upper and lower edges adjacent to the side edges. Simultaneously, the angle across the width of the third pursing strip 640 between the side edges 640s changes non-linearly at each line of weakness 641 which encourages the walls 601, 602 to separate (see Figures 21 and 22). In this embodiment, the ostomy appliance 600 is manufactured in a similar way to the first embodiment except that the distal wall 602 is additionally provided with the third pursing strip 640 in the position described above.

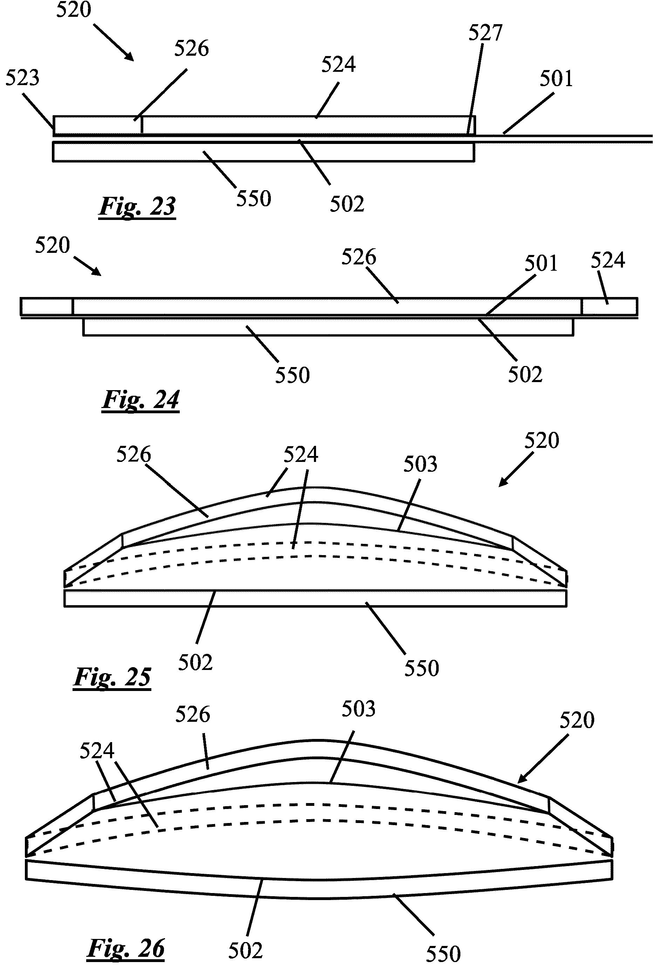

Referring to Figures 23-26 a fourth embodiment of an ostomy appliance 500 shares many of the same features of the first embodiment above and consequently only the differences are described below and like numerals are used to denote like features.

In this embodiment, the drain 520 comprises a third pursing strip 550. The third pursing strip 550 is the same size and shape as the first pursing strip 524 except that it is about 20% smaller than the width of the first pursing strip 524. Thus, the third pursing strip 550 is a narrow pursing strip. The third pursing strip 550 is arranged on the distal wall 502 at the distal end 523 of the drain.

Thus, in this embodiment, when the drain 520 is compressed, initially only the first and second pursing strips 524, 525 flex because they are wider than the third pursing strip 525. Then, after continued compression the third pursing strip 550 also flexes. This helps to open the drain 520 because it separates the opening action into two different phases where the forces on the walls of the drain are different in each phase. In addition, the curvature of the second and third pursing stripes 525, 550 are different from each other which helps to encourage the walls of the drain to separate.

In a variation on the third embodiment, the second pursing strip 525 may be omitted entirely and the drain 520 may have just two pursing strips: the first pursing strip 524 and third pursing strip 550.

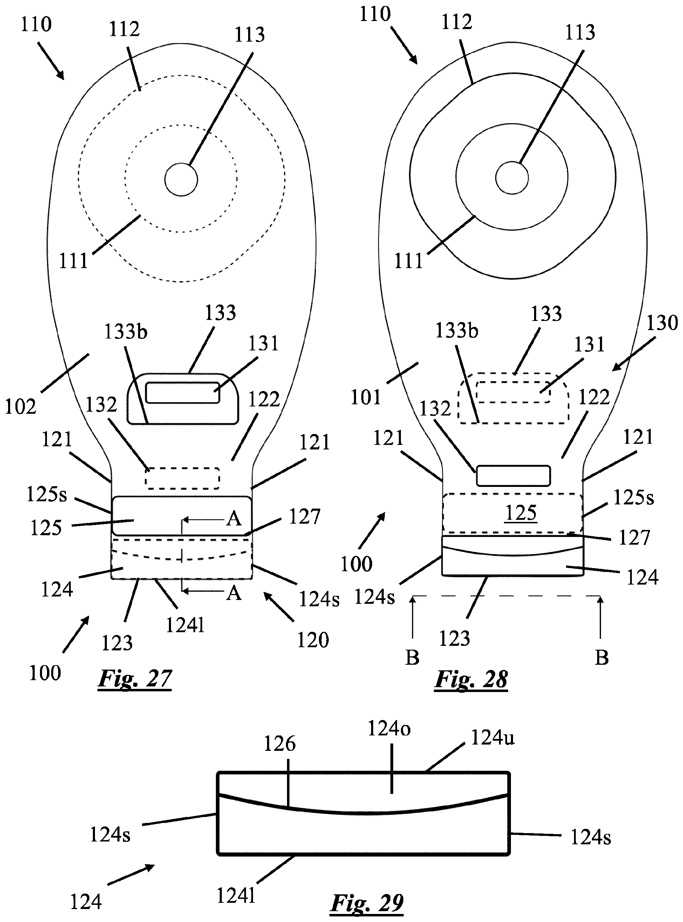

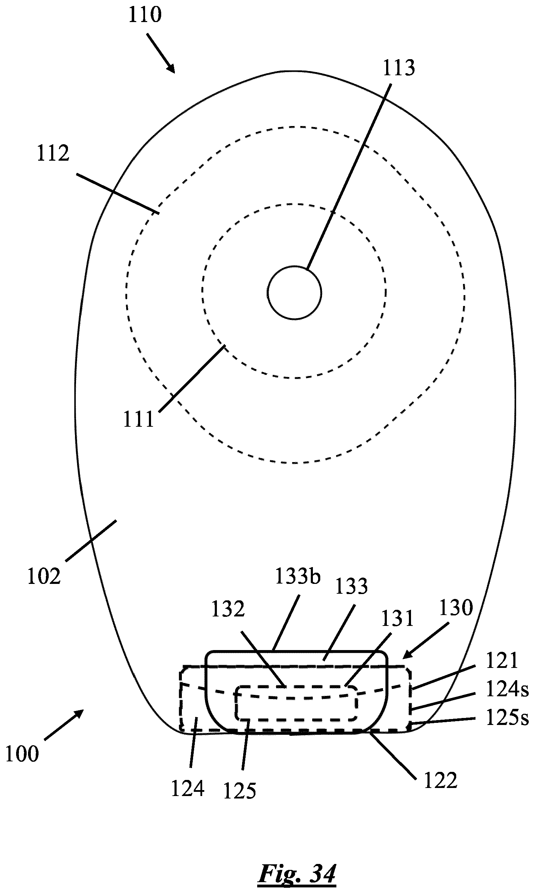

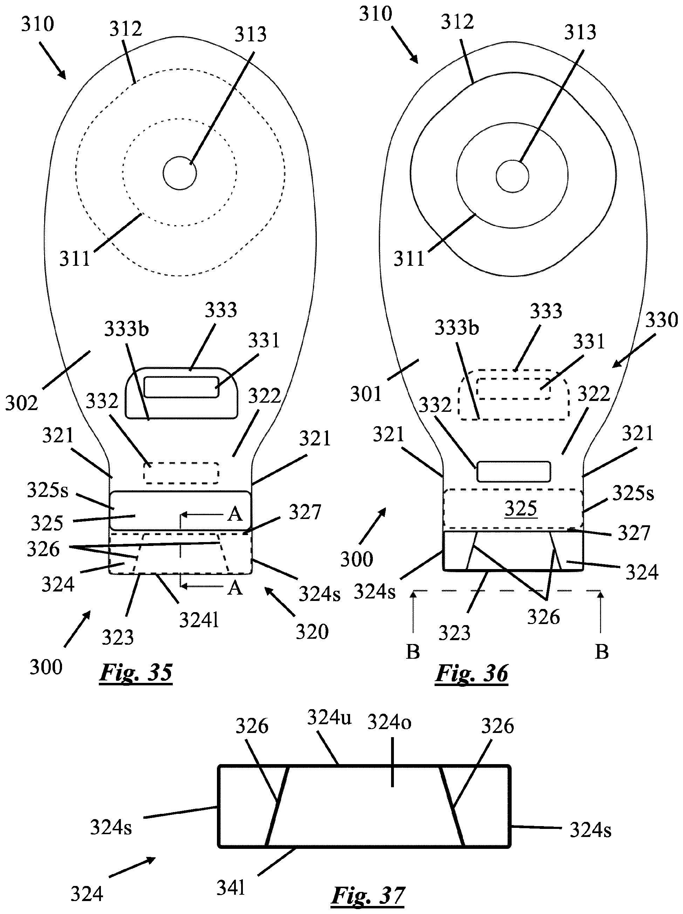

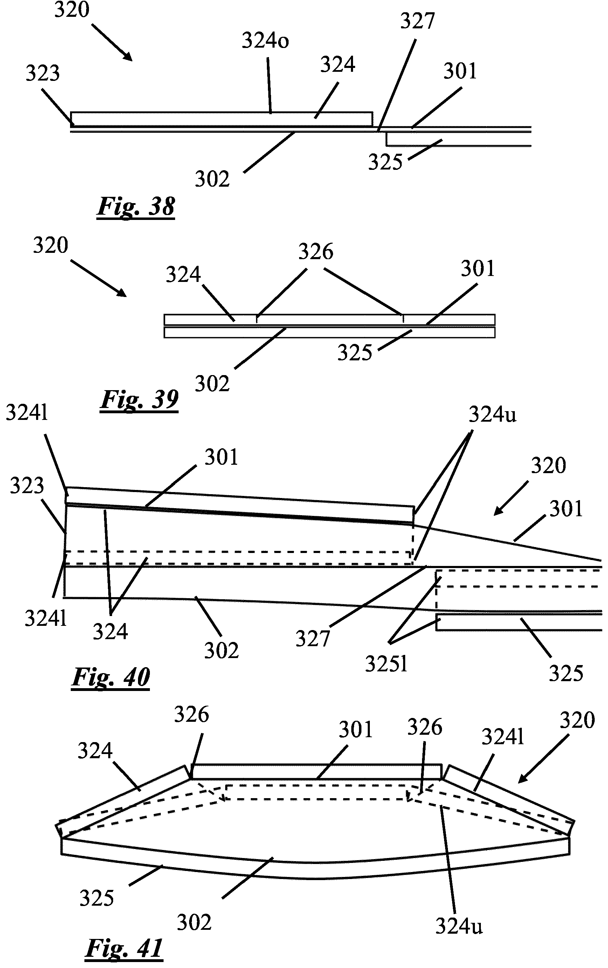

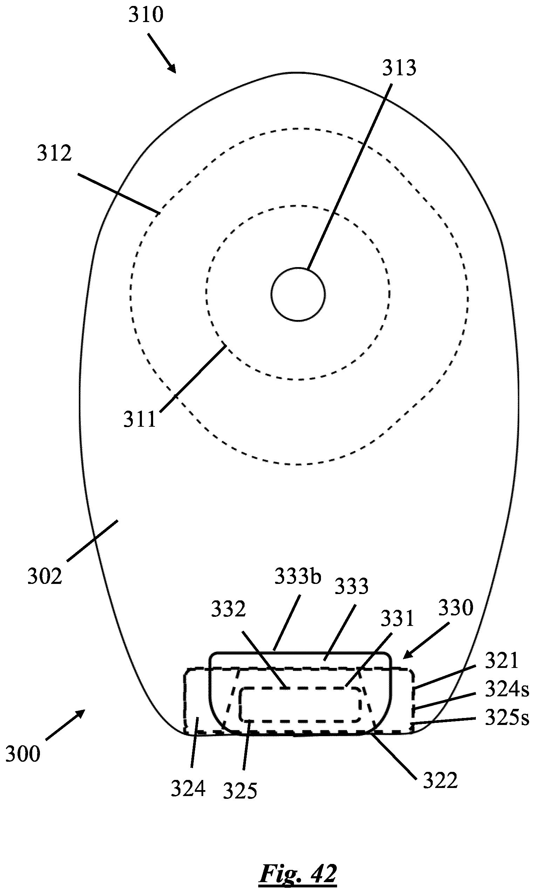

Referring to Figures 27 to 34 and Figures 35 to 42, a fifth embodiment of an ostomy appliance 100 and a sixth embodiment of an ostomy appliance 300 are shown respectively. The fifth and sixth embodiments are similar to the first embodiment above and so like numerals are used for corresponding features and only the differences are described below.

In both the fifth and sixth embodiments, the first pursing strip does not comprise a notch as in the first embodiment. Instead, the first pursing strip 124, 324 has one or more lines of weakness 126, 326.

The first pursing strip 124 of the fifth embodiment is equivalent to the third pursing strip of the second embodiment above and comprises a curved line of weakness 126 spanning completely between the side edges 124s of the first pursing strip 124.

The first pursing strip 324 of the sixth embodiment is equivalent to the third pursing strip of the third embodiment above and comprises two converging lines of weakness 326.

The first pursing strips 124, 324 of the fifth and sixth embodiments have all the features of the third pursing strips of the second and third embodiments above respectively, so are not described in detail.

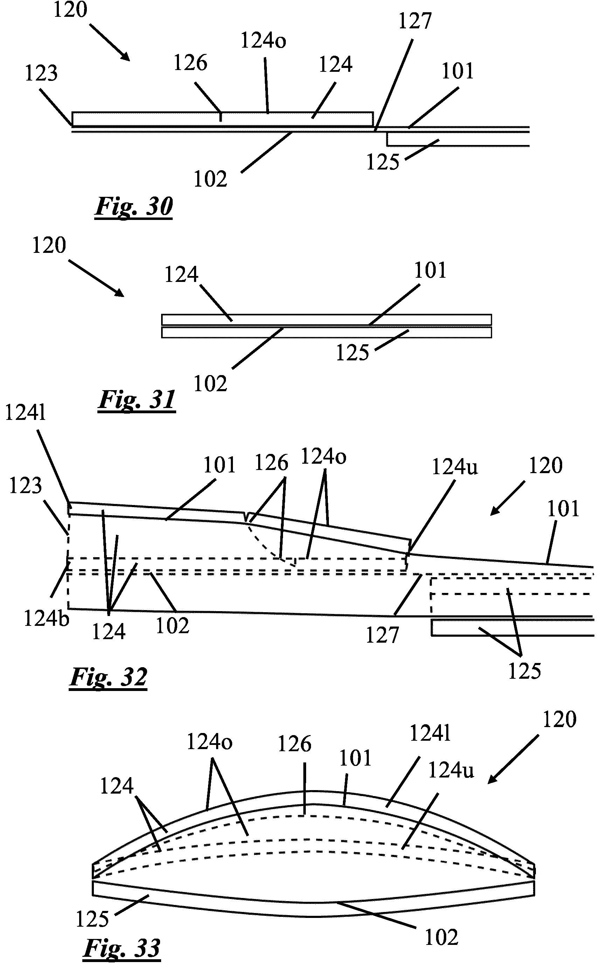

In the fifth and sixth embodiments, the first pursing strips 124, 324 operate as described above in relation to the second and third embodiments to assist opening the drain 120, 320 when compressed in a similar manner due to the lines of weakness 126, 326. As described above, the lines of weakness 126, 326 enable non-uniform compression and flexion of the pursing strips 124, 324 which assists in opening of the drain, this is shown in more detail in Figures 30 to 33 and 38 to 41.

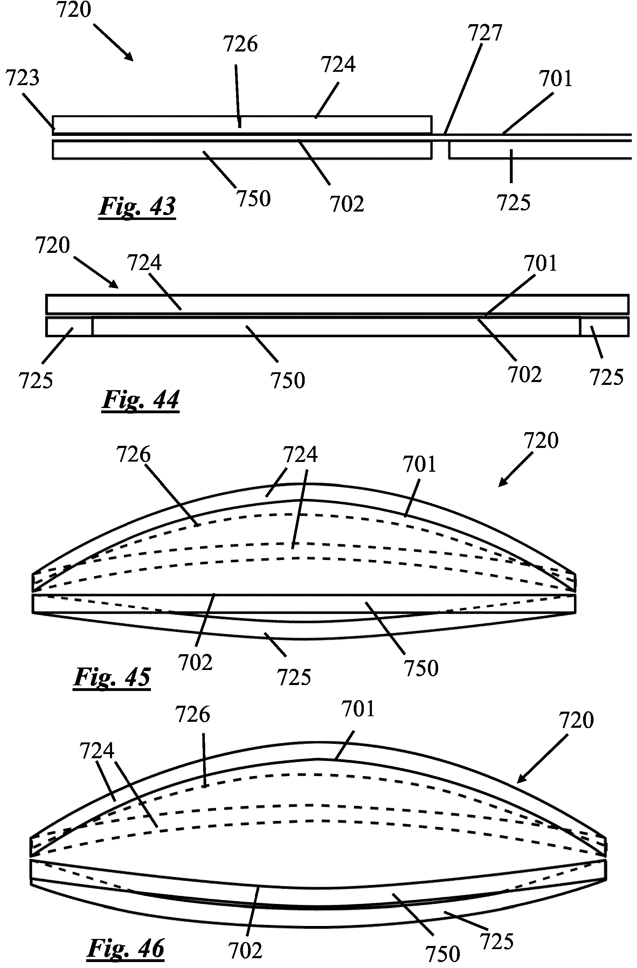

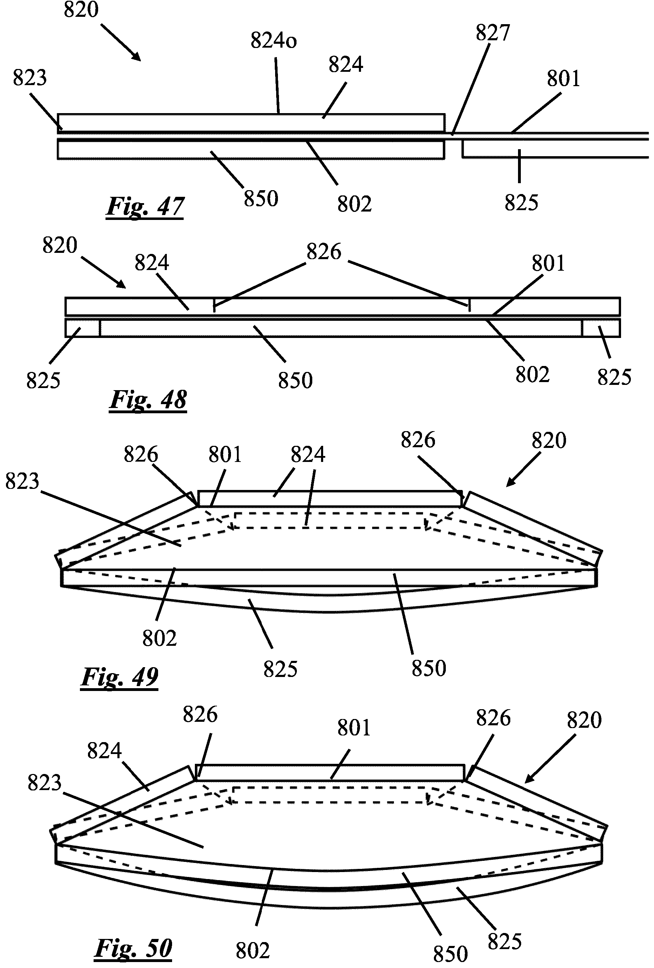

Referring to Figures 43 to 46 and Figures 47 to 50, a seventh embodiment of an ostomy appliance 700 and an eighth embodiment of an ostomy appliance 800 are shown respectively. The seventh and eighth embodiments are based on the fifth and sixth embodiments described above and so, like numerals are used to describe like features and only the differences are described.

The seventh and eighth embodiments additionally comprise a third pursing strip 750, 850 that is narrower than the first pursing strips 724, 824 arranged on the distal wall 702, 802 at the distal end 723, 823 of the drain. The third pursing strip 750, 850 is equivalent to the third pursing strip of the fourth embodiment described above and has all the same features.

As described above, the seventh and eighth embodiments thereby provide variations in which one or more lines of weakness on the first pursing strip are used in synergy with a narrower pursing strip to help assist in opening the drain.

The one or more embodiments are described above by way of example only. Many variations are possible without departing from the scope of protection afforded by the appended claims, for example all the sizes, shapes, and materials used are all exemplary.

The following numbered paragraphs additionally describe embodiments of the invention as set forth herein.
1. An ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a pursing strip of the one or more pursing strips comprises a transverse edge substantially perpendicular to the two lateral edges of the drain and the transverse edge comprises a notch spanning at least half of the transverse edge.
2. An ostomy appliance according to paragraph 1 wherein the notch has a width parallel to the transverse edge and a length perpendicular to its width and in a plane defined by the pursing strip, wherein the notch has an aspect ratio (width:length) of at least 7:1, and preferably an aspect ratio of at least 10:1.
3. An ostomy appliance according to paragraph 1 or 2 wherein the notch is arced and the arc radius of curvature is greater than the width of the notch.
4. An ostomy appliance according to any of paragraphs 1 to 3 wherein the pursing strip has a length parallel to the length of the notch and the length of the notch is less than half the length of the pursing strip.
5. An ostomy appliance according to any of paragraphs 1 to 4 further comprising a first pursing strip being the pursing strip comprising the notch, and a weakened pursing strip comprising a curved line of weakness.
6. An ostomy appliance according to paragraph 5 wherein the notch is arced and the curvature of the arc and line of weakness are opposing along the length of the drain.
7. An ostomy appliance according to any of paragraphs 1 to 6 wherein the drain comprises a proximal end to receive stomal output from the pouch and an opposite distal end, and the pursing strip is arranged at the distal end of the drain and the notch is provided in a distal transverse edge that is arranged at the distal end of the drain.
8. An ostomy appliance according to paragraph 7 wherein the distal end of the drain is straight and the notch exposes an end region of the distal end of the drain.
9. An ostomy appliance according to paragraph 8 wherein the end region is not directly attached to the one or more pursing strips.
10. An ostomy appliance according to any of paragraphs 1 to 9 wherein the notch is arranged at a midpoint along the transverse edge.
11. An ostomy appliance according to any of paragraphs 1 to 10 wherein the notch spans at least 60% of the transverse edge and preferably no more than 90% of the transverse edge.
12. An ostomy appliance according to any of paragraphs 1 to 11 wherein the one or more pursing strips comprises a pursing strip comprising one or more lines of weakness.
13. An ostomy appliance according to paragraph 12 wherein the pursing strip comprises two side edges corresponding to the two lateral edges of the drain and a line of weakness spanning between the side edges, and the line of weakness is curved.
14. An ostomy appliance comprising a pouch and a drain, wherein the drain is formed of two walls attached to one another along two lateral edges of the drain to define a channel therebetween and the drain comprises one or more pursing strips configured to separate the two walls of the drain when compressed in a direction perpendicular to the two lateral edges of the drain; wherein a pursing strip of the one or more pursing strips comprises two side edges corresponding to the two lateral edges of the drain and a line of weakness spanning between the side edges, and the line of weakness is curved.
15. An ostomy appliance according to paragraph 13 or 14 wherein the line of weakness extends from a point along one of the two side edges that is offset from a midpoint along the length of the side edge.
16. An ostomy appliance according to paragraph 15 wherein the line of weakness extends from a point along each of the two side edges that is offset from a midpoint along the length of the corresponding side edge.
17. An ostomy appliance according to any of paragraphs 13 to 16 wherein the pursing strip has a length defined parallel to the two side edges and a point along the line of weakness corresponds to a midpoint along the length of the pursing strip.
18. An ostomy appliance according to paragraph 17 wherein a midpoint along the line of weakness corresponds to a midpoint along the length of the pursing strip.
19. An ostomy appliance according to any of paragraphs 13 to 18 wherein the line of weakness is arranged in one half of the pursing strip.
20. An ostomy appliance according any of paragraphs 13 to 19 wherein the line of weakness is arced with a radius of curvature that is greater than the width of the pursing strip.
21. An ostomy appliance according to any of paragraphs 13 to 20 wherein the line of weakness is asymmetric about a line connecting the two side edges.
22. An ostomy appliance according to any of paragraphs 13 to 21 wherein the drain comprising a distal end for output of stomal effluent from the pouch, and the line of weakness is convex on a side facing the distal end of the drain.
23. An ostomy appliance according to any of paragraphs 13 to 22 wherein the line of weakness is curved to resists folding of the pursing strip by at least 90 degrees about the line of weakness.
24. An ostomy appliance according to any of paragraphs 13 to 23 wherein the drain is foldable about one or more fold lines into a closed configuration in which passage of stomal output through the drain is inhibited, and the line of weakness is provided on one side of at least one of the one or more fold lines.
25. An ostomy appliance according to any of paragraphs 12 to 24 wherein the drain comprising a distal end for output of stomal effluent from the pouch, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip.
26. An ostomy appliance comprising a pouch and a drain, the drain formed of proximal and distal walls each comprising two lateral edges, the drain comprising a distal end for output of stomal effluent from the pouch, wherein each lateral edge of one wall is attached to the corresponding lateral edge of the other wall to define a channel therebetween, the drain comprises two or more pursing strips configured to separate the walls of the drain when compressed in a direction perpendicular to the lateral edges of the drain; wherein a first pursing strip comprises an upper edge and a lower edge both perpendicular to the lateral edges and two converging lines of weakness spanning between the upper and lower edges; wherein the first pursing strip is arranged on the proximal wall at the distal end of the drain and a second pursing strip is arranged on the distal wall; and wherein, a longitudinal gap is provided between the upper edge of the first pursing strip and a lower edge of the second pursing strip.
27. An ostomy appliance according to paragraph 25 or 26 wherein the lines of weakness are arranged to converge at a point that is not on the first pursing strip.
28. An ostomy appliance according to paragraph 27 wherein the lines of weakness are arranged at an angle of at least 30 degrees from one another.
29. An ostomy appliance according to any of paragraphs 25 to 28 wherein the lines of weakness are each arranged at an angle by at least 15 degrees from a lateral edge of the drain.
30. An ostomy appliance according to any of paragraphs 25 to 29 wherein the lines of weakness are arranged to diverge in a direction towards the distal end of the drain.
31. An ostomy appliance according to any of paragraphs 25 to 30 wherein the lines of weakness are separated by at least half a width of the drain measured between the lateral edges of the drain.
32. An ostomy appliance according to any of paragraphs 25 to 31 wherein only the first pursing strip comprises one or more lines of weakness.
33. An ostomy appliance according to any of paragraphs 25 to 32 wherein the first pursing strip is the only pursing strip arranged on the proximal wall of the drain.
34. An ostomy appliance according to any of paragraphs 25 to 33 wherein the second pursing strip is the only pursing strip arranged on the distal wall of the drain.
35. An ostomy appliance according to any of paragraphs 25 to 34 further comprising a notched pursing strip, the notched pursing strip comprising a notch in a transverse edge of the notched pursing strip, the transverse edge being substantially parallel to the lateral edges of the drain.
36. An ostomy appliance according to any of paragraphs 13 to 35 wherein the line of weakness comprises a groove, or each of the lines of weakness comprises a groove.
37. An ostomy appliance according to any of paragraphs 1 to 36 wherein the drain has a width measured between the lateral edges of the drain and the pursing strip has a width substantially parallel to the width of the drain, wherein the width of the drain is the same as the width of the pursing strip.
38. An ostomy appliance according to any of paragraphs 1 to 37 wherein the one or more pursing strips comprises a first pursing strip comprising a notch or one or more lines of weakness and a second pursing strip, and the first and second pursing strips have equal widths.
39. An ostomy appliance according to any of paragraphs 1 to 38 wherein the one or more pursing strips comprises a first pursing strip comprising a notch or one or more lines of weakness, and a narrow pursing strip that has a smaller width than the first pursing strip.
40. An ostomy appliance according to any of paragraphs 1 to 39 wherein each of the one or more pursing strips is substantially flat.
41. A method of manufacturing an ostomy appliance comprising providing a pouch and two walls, forming a drain of the pouch by attaching the two walls to one another along two lateral edges of the drain to define a channel therebetween, and providing one or more pursing strips on the drain, wherein the one or more pursing strips are configured to separate the walls of the drain when compressed in a direction perpendicular to the two lateral edges of the drain, and a pursing strip of the one or more pursing strips comprises a transverse edge substantially perpendicular to the two lateral edges of the drain and the transverse edge comprises a notch spanning at least half of the transverse edge.
42. A method of manufacturing an ostomy appliance comprising providing a pouch and two walls, forming a drain of the pouch by attaching the two walls to one another along two lateral edges of the drain to define a channel therebetween, and providing one or more pursing strips on the drain, wherein the one or more pursing strips are configured to separate the walls of the drain when compressed in a direction perpendicular to the two lateral edges of the drain, the method comprising forming a line of weakness in a pursing strip of the one or more pursing strips between two side edges of the pursing strip that correspond to the lateral edges of the drain, wherein the line of weakness is curved.
43. A method of manufacturing an ostomy appliance comprising providing a pouch and proximal and distal walls, forming a drain of the pouch by attaching first and distal walls to one another along two lateral edges of the drain to define a channel therebetween, and providing two or more pursing strips on the drain, wherein the drain comprises a distal end for output of stomal effluent from the pouch, the two or more pursing strips are configured to separate the proximal and distal walls of the drain when compressed in a direction perpendicular to the two lateral edges of the drain, and each of the two or more pursing strips comprises an upper edge and a lower edge both perpendicular to the lateral edges of the drain, wherein the method comprises providing a first pursing strip, forming two converging lines of weakness in the first pursing strip that span between the upper and lower edges, arranging the first pursing strip on the proximal wall at the distal end of the drain and providing a second pursing strip arranged on the distal wall wherein, a longitudinal gap is provided between the upper edge of the first pursing strip and a lower edge of the second pursing strip.
44. A method of draining an ostomy appliance comprising a pouch and a drain, the drain comprising two walls sealed together along two lateral edges of the drain to define a channel therebetween, the method comprising compressing a pursing strip of the drain in a direction perpendicular to the two lateral edges of the drain to separate the two walls, wherein the pursing strip comprises a transverse edge substantially perpendicular to the two lateral edges of the drain and the transverse edge comprises a notch spanning at least half of the transverse edge.
45. A method of draining an ostomy appliance comprising a pouch and a drain, the drain comprising two walls sealed together along two lateral edges of the drain to define a channel therebetween, the method comprising compressing a pursing strip of the drain in a direction perpendicular to the two lateral edges of the drain to separate the two walls, wherein the pursing strip comprises a line of weakness spanning between two side edges of the pursing strip that correspond to the two lateral edges of the drain, wherein the line of weakness is curved.
46. A method of draining an ostomy appliance comprising a pouch and a drain, the drain comprising proximal and distal walls sealed together along two lateral edges of the drain to define a channel therebetween, wherein the drain comprises two or more pursing strips each comprising an upper edge and a lower edge both of which are perpendicular to the lateral edges of the drain, the method comprising compressing a first pursing strip of the two or more pursing strips in a direction perpendicular to the two lateral edges of the drain to separate the proximal and distal walls, wherein the first pursing strip comprises two converging lines of weakness spanning between the upper and lower edges of the first pursing strip, wherein the first pursing strip is arranged on the proximal wall at the distal end of the drain, a second pursing strip is arranged on the distal wall and a longitudinal gap is provided between the upper edge of the first pursing strip and a lower edge of the second pursing strip.
47. A method according to paragraphs 41 to 46 wherein the ostomy appliance is the ostomy appliance according to any of paragraphs 1 to 40.

## Claims

1. An ostomy appliance comprising a pouch and a drain, the drain formed of two walls attached to one another along two lateral edges of the drain to define a channel therebetween, the drain comprises one or more pursing strips configured to separate the walls of the drain when compressed in a direction perpendicular to the two lateral edges of the drain, wherein a pursing strip of the one or more pursing strips comprises a transverse edge substantially perpendicular to the two lateral edges of the drain and the transverse edge comprises a notch spanning at least half of the transverse edge.

2. An ostomy appliance according to claim 1 wherein the notch has a width parallel to the transverse edge and a length perpendicular to its width and in a plane defined by the pursing strip, wherein the notch has an aspect ratio (width:length) of at least 7:1, and preferably an aspect ratio of at least 10:1.

3. An ostomy appliance according to claim 1 or 2 wherein the notch is arced and the arc radius of curvature is greater than the width of the notch.

4. An ostomy appliance according to any preceding claim wherein the pursing strip has a length parallel to the length of the notch and the length of the notch is less than half the length of the pursing strip.

5. An ostomy appliance according to any preceding claim further comprising a first pursing strip being the pursing strip comprising the notch, and a weakened pursing strip comprising a curved line of weakness.

6. An ostomy appliance according to claim 5 wherein the notch is arced and the curvature of the arc and line of weakness are opposing along the length of the drain.

7. An ostomy appliance according to any preceding claim wherein the drain comprises a proximal end to receive stomal output from the pouch and an opposite distal end, and the pursing strip is arranged at the distal end of the drain and the notch is provided in a distal transverse edge that is arranged at the distal end of the drain.

8. An ostomy appliance according to claim 7 wherein the distal end of the drain is straight and the notch exposes an end region of the distal end of the drain.

9. An ostomy appliance according to claim 8 wherein the end region is not directly attached to the one or more pursing strips.

10. An ostomy appliance according to any preceding claim wherein the notch is arranged at a midpoint along the transverse edge.

11. An ostomy appliance according to any preceding claim wherein the notch spans no more than 90% of the transverse edge.

12. An ostomy appliance according to any preceding claim wherein the one or more pursing strips comprises a pursing strip comprising one or more lines of weakness.

13. An ostomy appliance according to claim 12 wherein the pursing strip comprises two side edges corresponding to the two lateral edges of the drain and a line of weakness spanning between the side edges, and the line of weakness is curved.

14. An ostomy appliance according to claim 12 or 13 wherein the pursing strip comprises an upper edge and a lower edge both perpendicular to the lateral edges and two converging lines of weakness spanning between the upper and lower edges.

15. An ostomy appliance according to any preceding claim wherein the one or more pursing strips comprises a first pursing strip comprising a notch or one or more lines of weakness, and a narrow pursing strip that has a smaller width than the first pursing strip.
